# EUROPEAN PATENT APPLICATION

(11) **EP 4 806 893 A1**
(43) Date of publication of application: **16.09.2026**
(21) Application number: 24910300.3
(22) Date of filing: 01.11.2024
(51) Int. Cl.: C07D 413/12, C09K 11/06, H10K 85/60, H10K 50/11

(54) **BENZONAPHTHOFURAN-CONTAINING OXAZOLE ORGANIC COMPOUND AND USE THEREOF**

(30) Priority: 29.12.2023 CN 202311868168; 01.03.2024 CN 202410240330
(71) Applicant: Ningbo Lumilan Advanced Materials Co., Ltd., Ningbo, Zhejiang 315040 (CN)
(72) Inventor: WEI, Ting-wei, Ningbo, Zhejiang 315040 (CN); TANG, Cong, Ningbo, Zhejiang 315040 (CN); LIU, Fengjiao, Ningbo, Zhejiang 315040 (CN); SUN, Shuangshuang, Ningbo, Zhejiang 315040 (CN); DING, Huanda, Ningbo, Zhejiang 315040 (CN); XIAO, Yang, Ningbo, Zhejiang 315040 (CN); CHEN, Zhikuan, Ningbo, Zhejiang 315040 (CN)
(74) Representative: J A Kemp LLP
(86) International application number: PCT/CN2024/129250
(87) International publication number: WO 2025/139362

(57) **Abstract**

The present application relates to the technical field of display, and in particular to a benzonaphthofuran-containing oxazole organic compound and a use thereof. The benzonaphthofuran-containing oxazole organic compound provided by the present application has a structure as shown below: 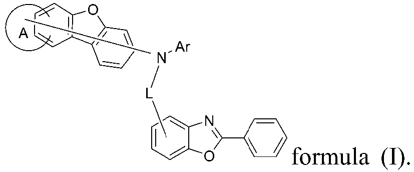 An organic light-emitting device containing the benzonaphthofuran-containing oxazole organic compound can have relatively low driving voltage, relatively high light-emitting efficiency and relatively long service life.

## Description

### CROSS-REFERENCE TO RELATED APPLICATIONS

The present application claims priority to Chinese Patent Application No. 202311868168.X, filed to the China National Intellectual Property Administration on December 29, 2023, entitled "BENZONAPHTHOFURAN-CONTAINING OXAZOLE ORGANIC COMPOUND AND USE THEREOF", and priority to Chinese Patent Application No. 202410240330.1, filed to China National Intellectual Property Administration on March 1, 2024, entitled "BENZONAPHTHOFURAN-CONTAINING OXAZOLE ORGANIC COMPOUND AND USE THEREOF", the entire contents of which are incorporated herein by reference.

### TECHNICAL FIELD

The present application relates to the field of display technology, and in particular to a benzonaphthofuran-containing oxazole organic compound and use thereof.

### BACKGROUND

Organic electroluminescent devices (OLED) are devices that convert electrical energy into light by applying electricity to organic electroluminescent materials, and generally have a structure that includes an anode, a cathode, and an organic layer disposed between the anode and cathode. The organic layer of an organic EL device can be composed of a hole injection layer, a hole transport layer, an electron blocking layer, a light-emitting layer (including host materials and doping materials), an electron buffer layer, a hole blocking layer, an electron transport layer, an electron injection layer, and so on. Moreover, the materials used for the organic layer are classified by their functions as hole injection materials, hole transport materials, electron blocking materials, light-emitting materials, electron buffer materials, hole blocking materials, electron transport materials, electron injection materials, and so on. In the organic EL device, due to the application of voltage, holes are injected into the light-emitting layer from the anode, electrons are injected into the light-emitting layer from the cathode, and high-energy excitons are formed through the recombination of holes and electrons. Through this energy, the organic luminescent compound reaches an excited state and emits light due to the energy generated by the excited state of the organic luminescent compound transitioning to the ground state.

The most important factor determining the luminous efficiency in organic EL devices is the luminescent material. Luminescent materials must have high quantum efficiency, as well as high electron and hole mobility, and the formed luminescent material layer must be uniform and stable. Luminescent materials can be classified into blue light emitting materials, green light emitting materials, red light emitting materials, as well as additional yellow light emitting materials or orange light emitting materials, based on the color of their light emission. In addition, luminescent materials can be classified into host materials and dopant materials based on their functions.

However, the stability of existing organic electroluminescent materials is relatively low, and the matching degree between HOMO, LUMO energy levels and adjacent energy levels is relatively poor, resulting in the problem of unbalanced carrier mobility, which in turn leads to the problems of relatively high driving voltage, relatively low luminous efficiency, and relatively short lifetime of organic electroluminescent devices containing this organic electroluminescent material, seriously limiting the application of organic electroluminescent devices.

### SUMMARY OF THE INVENTION

The object of the present application is to overcome the problems of relatively high driving voltage, relatively low luminous efficiency, and relatively short lifetime of organic electroluminescent devices containing this organic electroluminescent material, caused by the problem of unbalanced carrier mobility due to the relatively low stability of existing organic electroluminescent materials, and the matching degree between HOMO, LUMO energy levels and adjacent energy levels being relatively poor. Therefore, a benzonaphthofuran-containing oxazole organic compound and use thereof are provided.

In the present application, the definition of substituent terms is as follows.

As used in the present application, the term "halogen" may include fluorine, chlorine, bromine or iodine.

As used in the present application, the term "C1-C30 alkyl" refers to a monovalent substituent derived from a straight chain or branched chain saturated hydrocarbon having 1 to 30 carbon atoms, examples of which include but are not limited to methyl, ethyl, propyl, isobutyl, sec-butyl, tert-butyl, pentyl, isopentyl and hexyl.

As used in the present application, the term "C3-C30 cycloalkyl" refers to a group derived from a monocyclic hydrocarbon or a polycyclic hydrocarbon having 1 to 30 ring main chain carbon atoms, and the cycloalkane may include cyclopropyl, cyclobutyl, adamantyl, and so on.

In the present application, aryl and arylene include monocyclic, polycyclic or fused ring aryl groups, the rings may be interrupted by short non-aromatic units among the rings and may contain spiro structures. Aryl includes, but is not limited to phenyl, biphenyl, terphenyl, naphthyl, phenanthryl, anthryl, fluorenyl, spirobifluorenyl, and so on, and arylene include but are not limited to phenylene, biphenylene, terphenylene, naphthylene, phenanthrene, anthrylene, fluorenylene, spirobifluorenylene groups, and so on.

In the present application, heteroaryl and heteroarylene include monocyclic, polycyclic, or fused ring heteroaryl groups, the rings may be interrupted by short non-aromatic units among the rings, and the heteroatoms include nitrogen, oxygen, and sulfur. Heteroaryl includes, but is not limited to furnan, thiophene, pyrrole, imidazole, pyrazole, thiazole, thiadiazole, isothiazole, isoxazole, oxazole, oxadiazole, triazine, tetrazine, triazole, tetrazole, furazan, pyridine, pyrazine, pyrimidine, pyridazine, benzofuran, benzothiophene, isobenzofuran, dibenzofuran, dibenzothiophene, benzimidazole, benzothiazole, benzisothiazole, benzisoxazole, benzoxazole, isoindole, indole, indazole, benzothiadiazole, quinoline, isoquinoline, cinnoline, quinazoline, quinoxaline, carbazole, phenoxazine, phenothiazine, phenanthridine, benzodioxolane, dihydroacridine, and derivatives thereof. Heteroarylene includes, but is not limited to furylidene, phenylthioylidene, pyrrolylidene, imidazolylidene, pyrazolylidene, thiazolylidene, thiadiazolylidene, isothiazolylidene, isoxazolylidene, oxazolylidene, oxadiazolylidene, triazinlidene, tetrazinylidene, triazolylidene, tetrazolylidene, furazanylidene, pyridylidene, pyrazinylidene, pyrimidinylidene, pyridazinylidene, benzofurylidene, benzothienylidene, isobenzofurylidene, dibenzofurylidene, dibenzothienylidene, benzimidazolylidene, benzothiazolylidene, benzisothiazolylidene, benzisoxazolylidene, benzoxazolylidene, isoindolylidene, indolylidene, indazolylidene, benzothiadiazolylidene, quinolinylidene, isoquinolinylidene, cinnolinylidene, quinazolinylidene, quinoxalinylidene, carbazolylidene, phenoxazinylidene, phenothiazinylidene, phenanthridinylidene, benzodioxolylidene, acridinylidene, and derivatives thereof.

As used in the present application, the term "substituted" means that a hydrogen atom in a compound is replaced by another substituent. This position is not limited to a specific location as long as the hydrogen at the position can be replaced by a substituent. When two or more substituents are present, the two or more substituents may be the same or different.

As used in the present application, unless otherwise specified, hydrogen atoms include protium, deuterium and tritium.

In the present application, the range of the number of carbon atoms is defined in the definition of the group, and the number of carbon atoms is any integer within the defined range. For example, for C6-C30 aryl, the number of carbon atoms representing the aryl can be any integer within the range of 6-60, such as 6, 8, 10, 13, 15, 17, 20, 22, 25, or 30, and so on. In the present application, 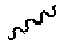 refers to a connection bond.

The technical solutions adopted in the present application are as follows.

The present application provides a benzonaphthofuran-containing oxazole organic compound, the oxazole organic compound has a structure represented by Formula (1):

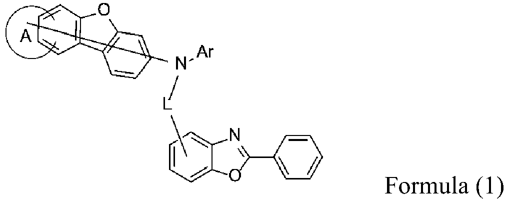

In Formula (1), ring A is a benzene ring;
Ar is selected from the group consisting of substituted or unsubstituted C6-C60 aryl, substituted or unsubstituted C6-C60 arylamine, substituted or unsubstituted C3-C60 heteroarylamine, and substituted or unsubstituted C3-C30 heteroaryl;
L is selected from the group consisting of substituted or unsubstituted C6-C30 arylene, and substituted or unsubstituted C3-C30 heteroarylene;
the substituents in the substituted C6-C60 aryl, substituted C6-C60 arylamine, substituted C3-C60 heteroarylamine, substituted C3-C30 heteroaryl, substituted C6-C30 arylene, and substituted C3-C30 heteroarylene are each independently selected from the group consisting of deuterium, halogen, cyano, C1-C12 alkyl, C3-C12 cycloalkyl, C6-C30 aryl, C3-C30 heteroaryl, C6-C60 arylamine, and C3-C60 heteroarylamine, or a combination of at least two thereof.

It can be understood that, 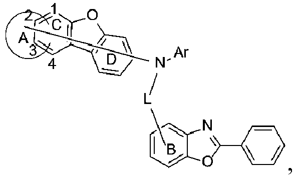 ring A in Formula (1) can be fused with ring C through positons 1, 2; 2, 3; 3, 4; N can be directly connected to any substitutive position in ring A, ring C and ring D; and L can be connected to any substitutive position in ring B.

Optionally, Ar is selected from the group consisting of substituted or unsubstituted C6-C25 aryl, substituted or unsubstituted C6-C25 arylamine, substituted or unsubstituted C3-C25 heteroarylamine, and substituted or unsubstituted C3-C20 heteroaryl;
wherein the substituents in the substituted C6-C25 aryl, substituted C6-C25 arylamine, substituted C3-C25 heteroarylamine, and substituted C3-C20 heteroaryl are each independently selected from the group consisting of deuterium, halogen, cyano, C1-C12 alkyl, C3-C12 cycloalkyl, C6-C30 aryl, C3-30 heteroaryl, C6-C60 arylamine and C3-C60 heteroarylamine, or a combination of at least two thereof;
optionally, Ar is selected from substituted or unsubstituted B group, and B group is selected from the group consisting of phenyl, naphthyl, biphenyl, phenanthryl, fluoranthenyl, chrysenyl, terphenyl, triphenylenyl, phenalenyl, phenylnaphthyl, naphthylphenyl, dimethylfluorenyl, phenylmethylfluorenyl, diphenylfluorenyl, pyridyl, pyridylphenyl, phenylpyridyl, spirobifluorenyl, benzodimethylfluorenyl, benzodiphenylfluorenyl, benzospirobifluorenyl, dibenzofuryl, benzonaphthofuryl, benzonaphthothienyl, spiro[fluorene-9,9'-xanthene]-yl, phenylmethylfluorenyl, dinaphthofuranyl, dinaphthothiophenyl, dibenzothiophenyl, and N, N-diphenylanilino;
wherein the substituents of the substituted B are selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof.

Optionally, Ar is selected from the group consisting of phenyl, naphthyl, biphenyl, chrysenyl, phenanthryl, terphenyl, phenylnaphthyl, naphthylphenyl, dimethylfluorenyl, diphenylfluorenyl, spirobifluorenyl, phenalenyl, dibenzofuryl, benzonaphthofuryl and N, N-diphenylanilino.

Optionally, L is selected from the group consisting of substituted or unsubstituted C6-C15 arylenes; wherein the substituents in the substituted C6-C15 arylene are each independently selected from the group consisting of deuterium, halogen, and C1-C62 alkyl, or a combination of at least two thereof;
optionally, L is selected from the group consisting of phenylene, biphenylene, and naphthylene; and
optionally, L is selected from the group consisting of phenylene and naphthylene.

Optionally, Formula (1) is selected from one of the structures represented by Formulas 1-1 to 1-6 below: 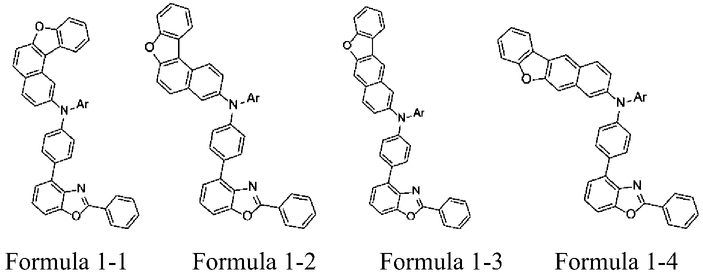 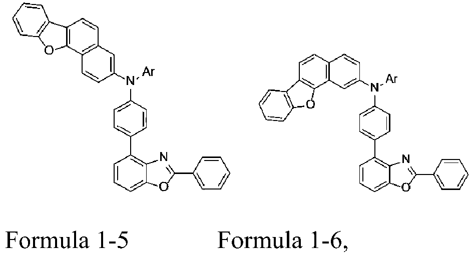 wherein the definition of Ar is the same as those mentioned above.

Optionally, Formula (1) is selected from one of the structures shown in Formulas 1-7 to 1-15 below: 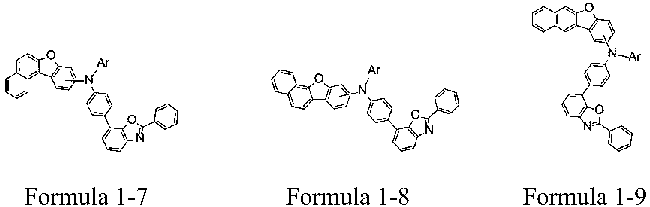 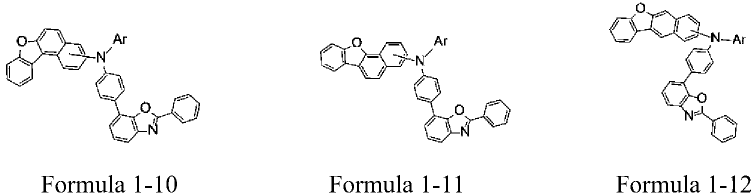 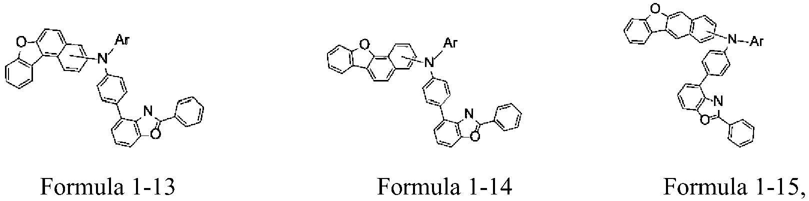 wherein the definition of Ar is the same as those mentioned above.

Optionally, the benzonaphthofuran-containing oxazole organic compound is selected from any one of N-1 to N-208:

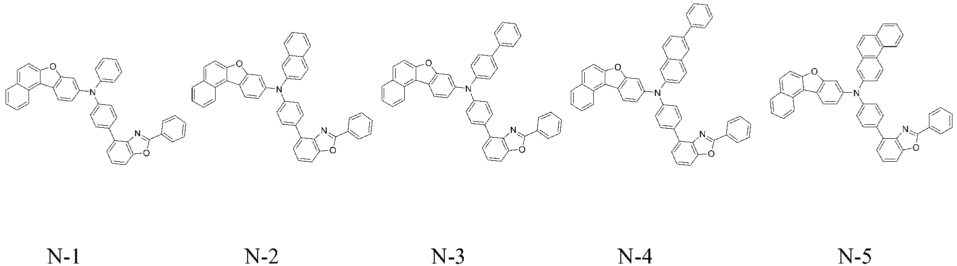

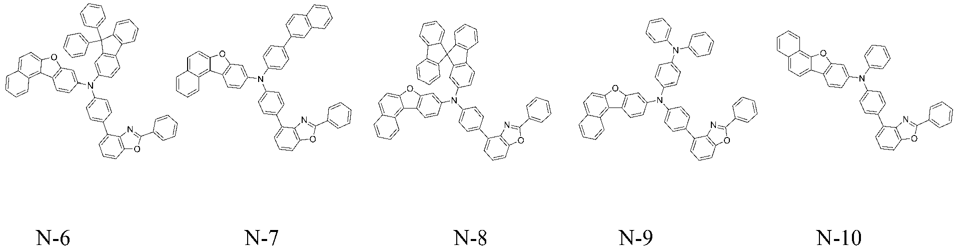

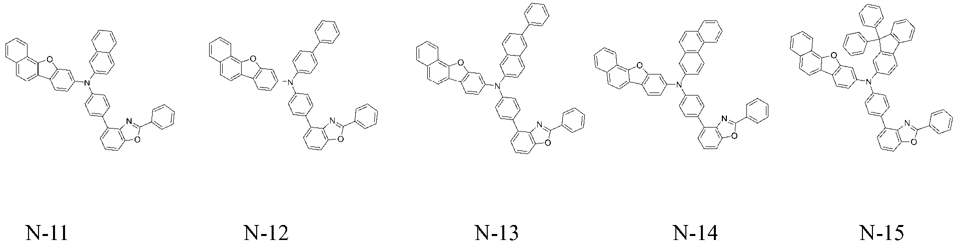

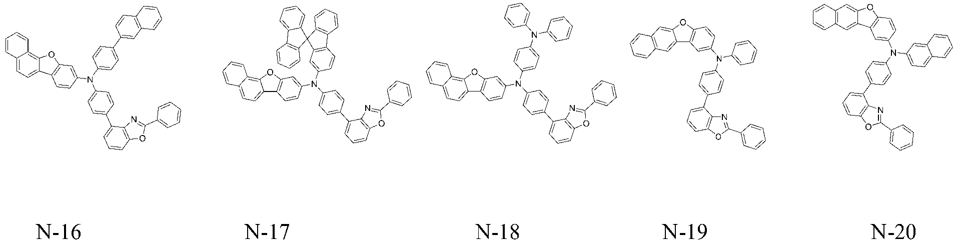

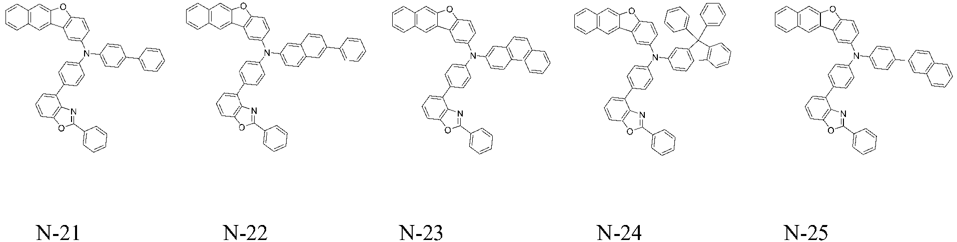

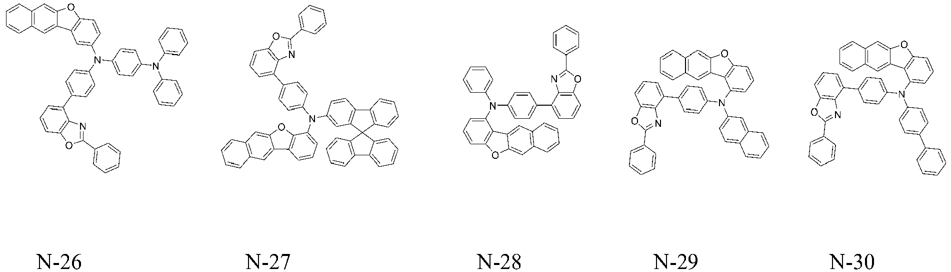

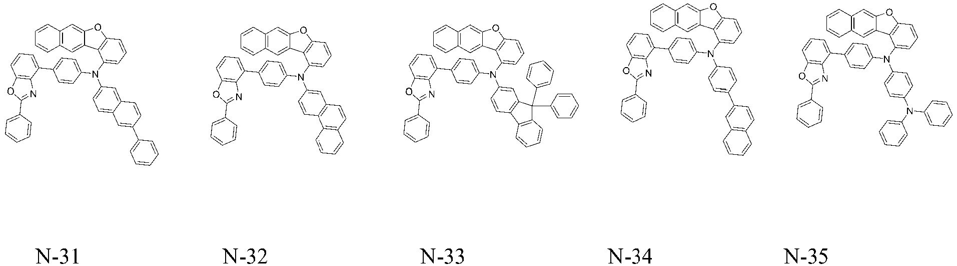

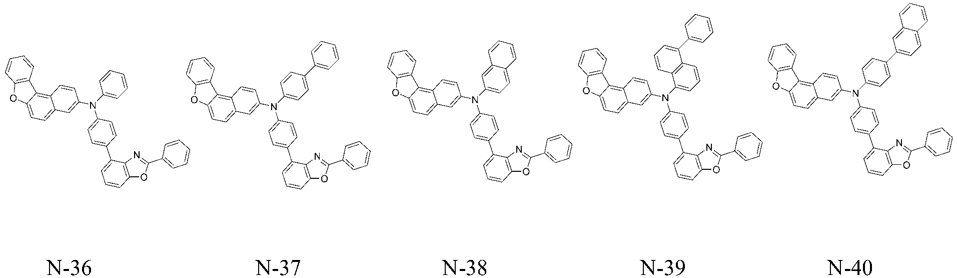

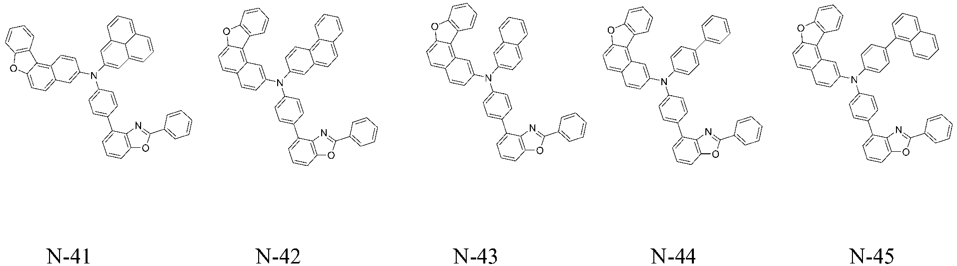

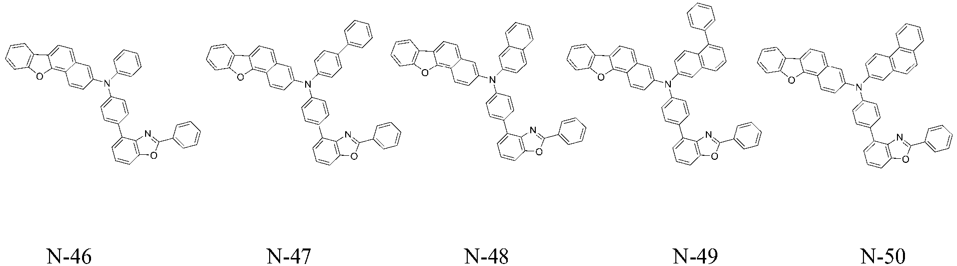

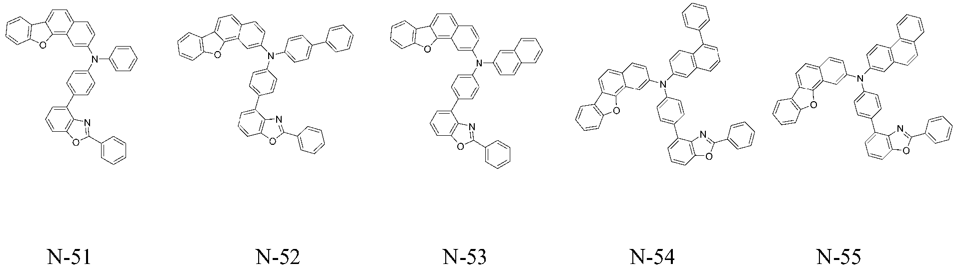

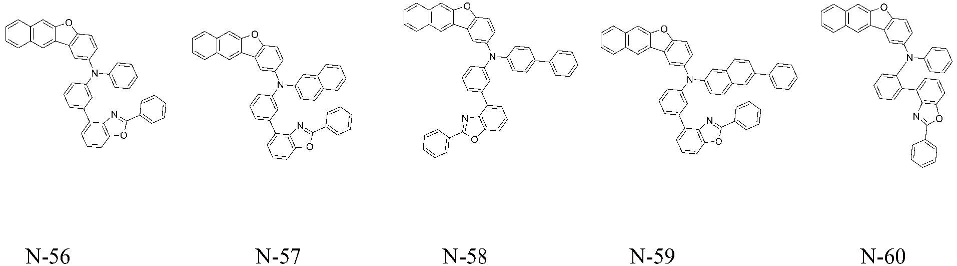

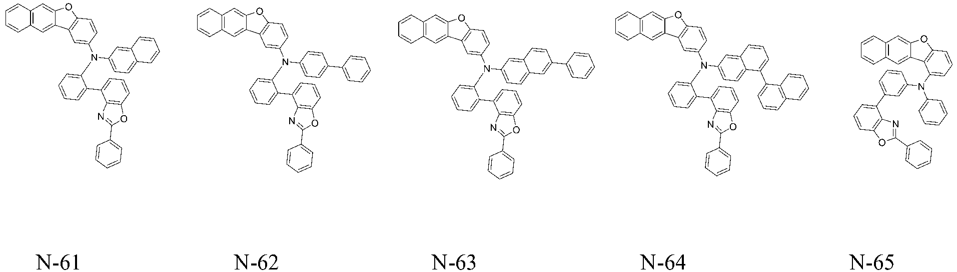

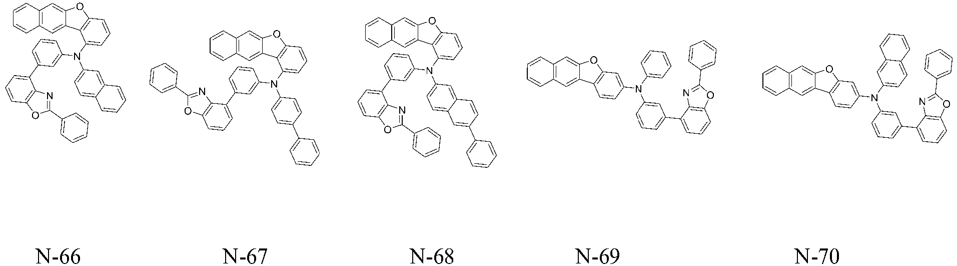

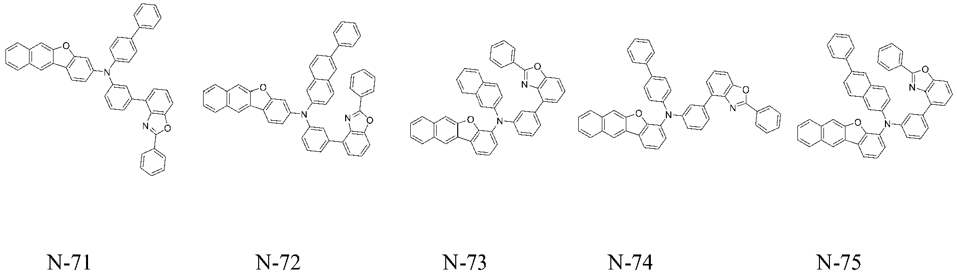

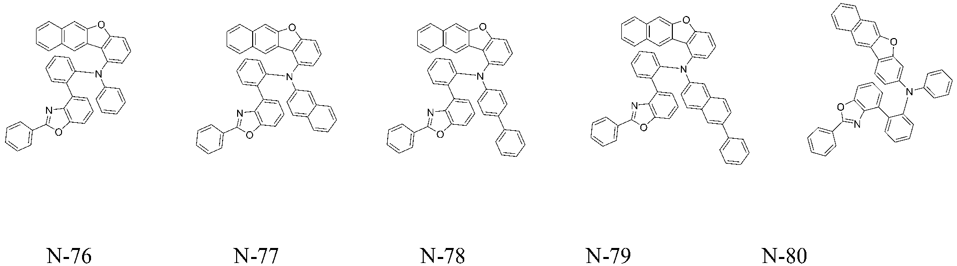

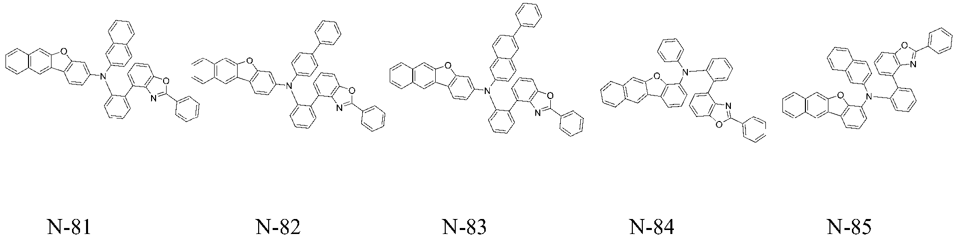

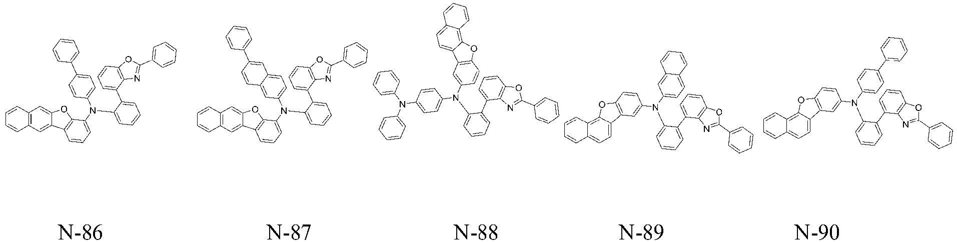

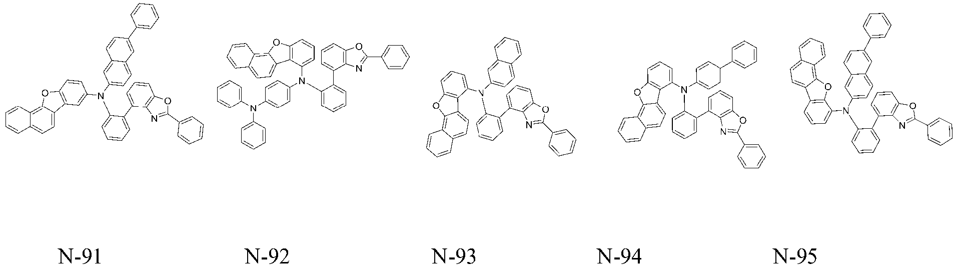

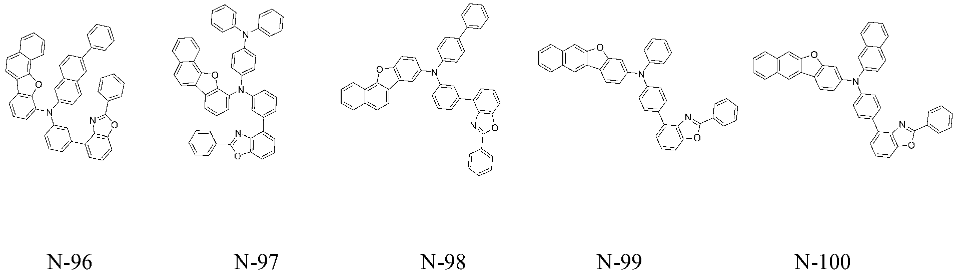

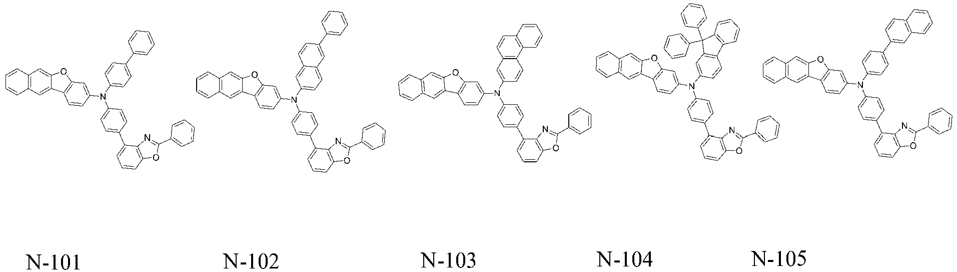

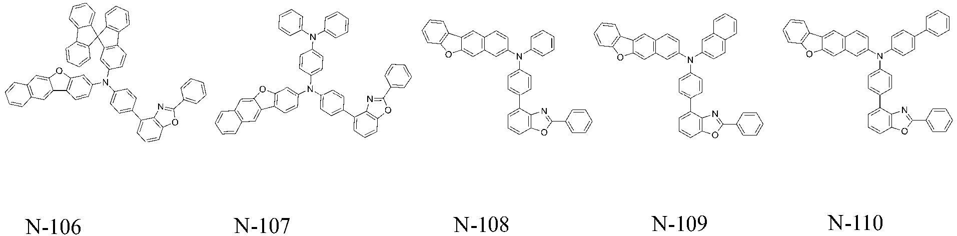

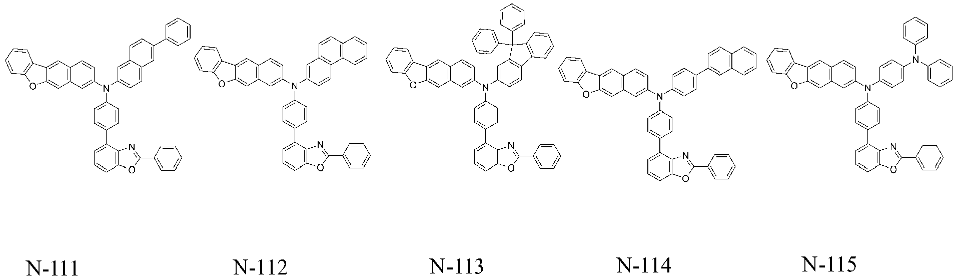

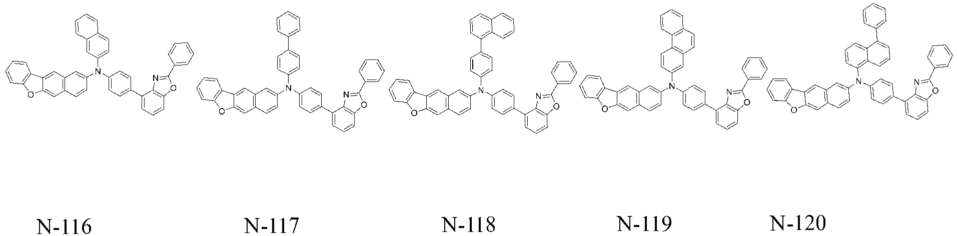

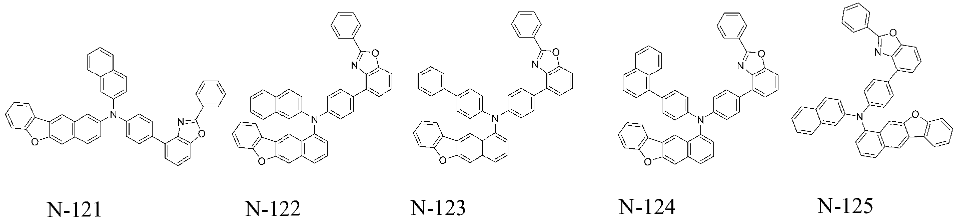

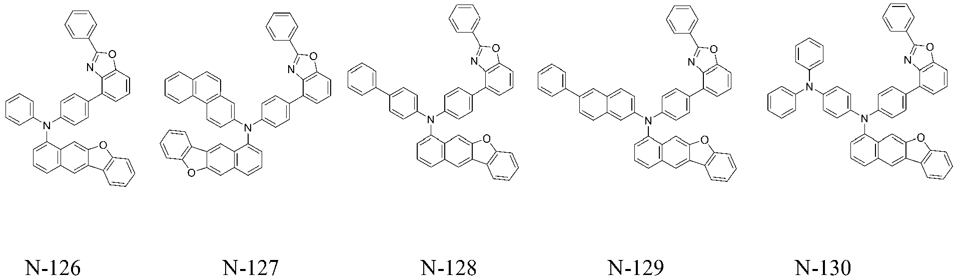

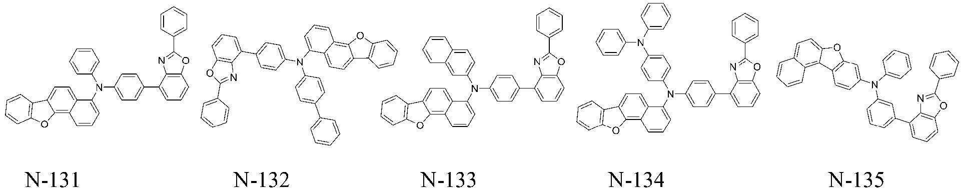

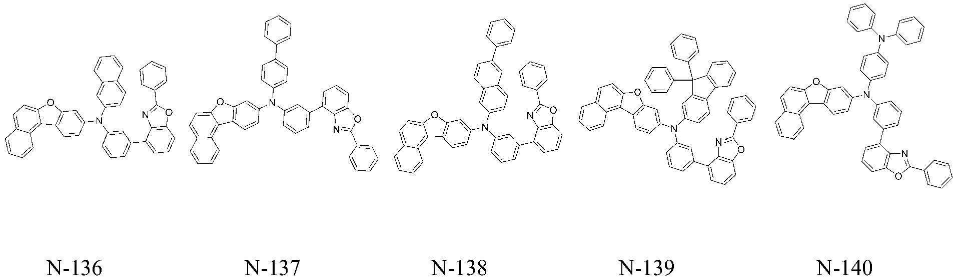

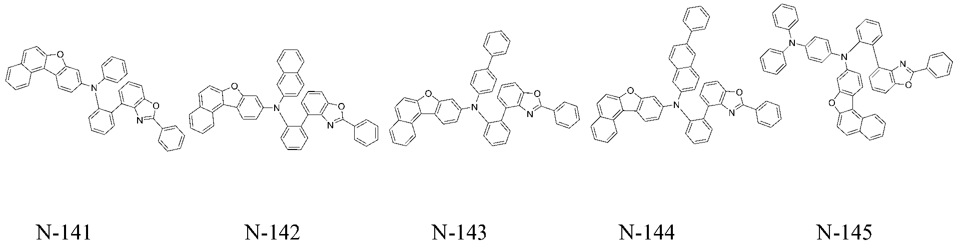

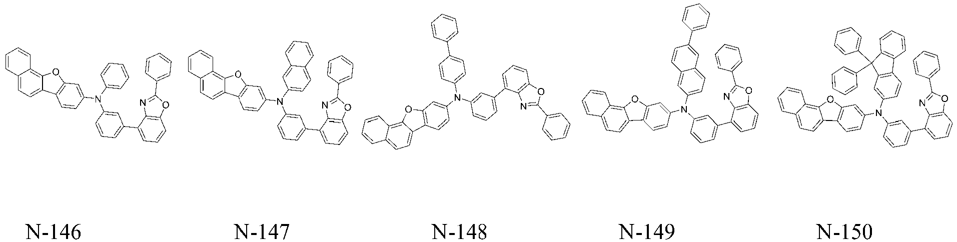

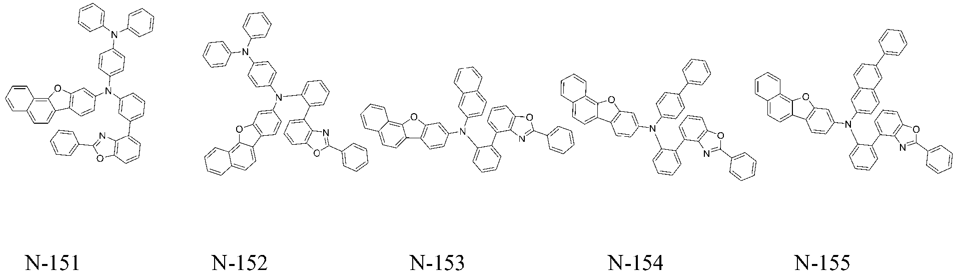

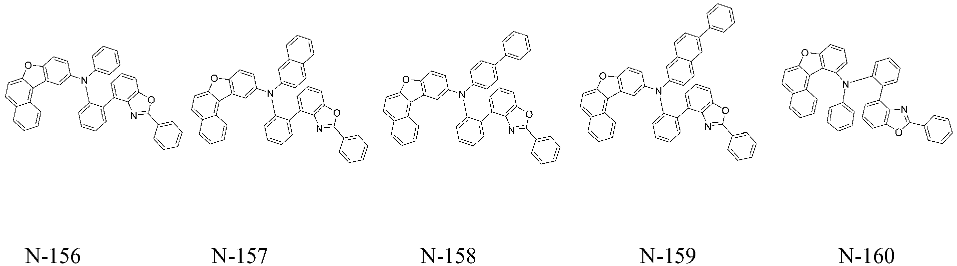

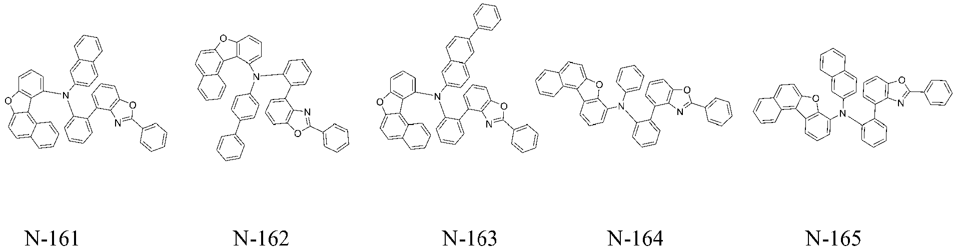

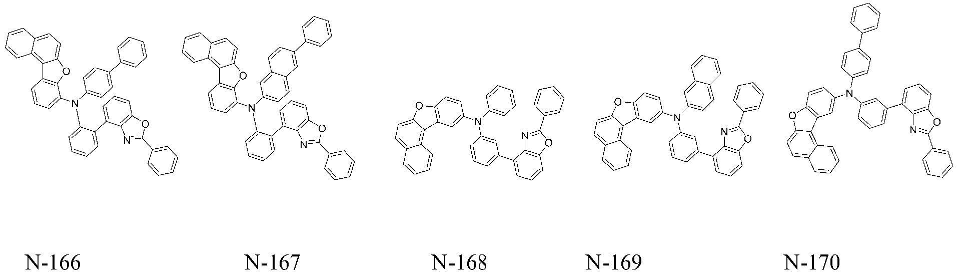

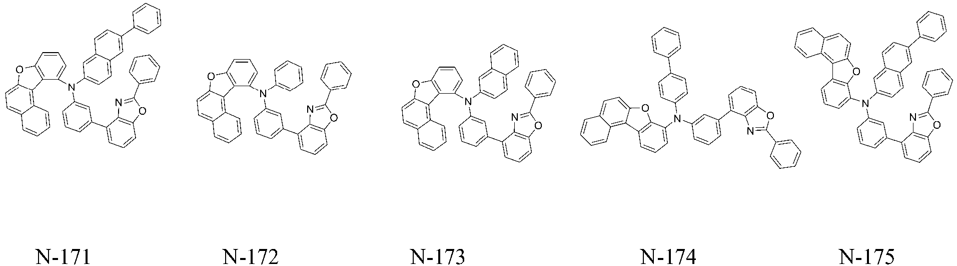

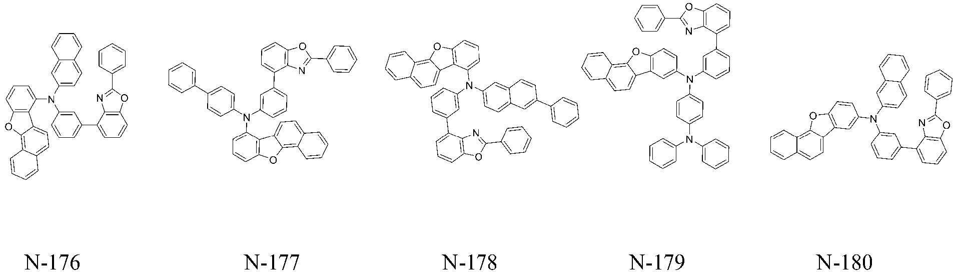

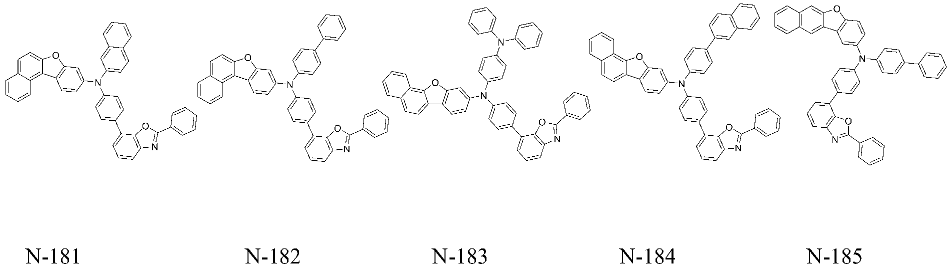

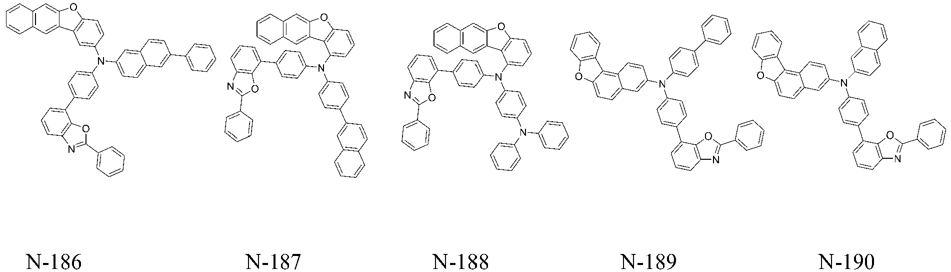

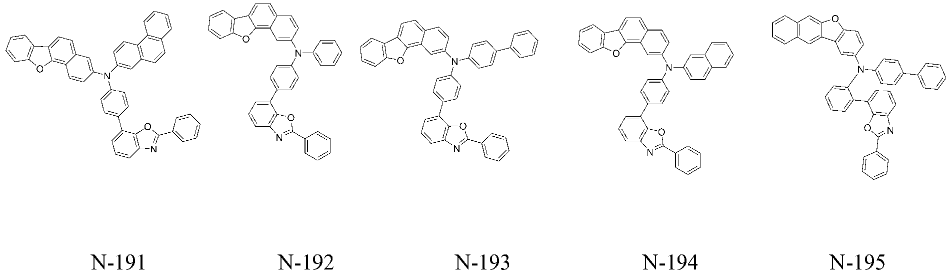

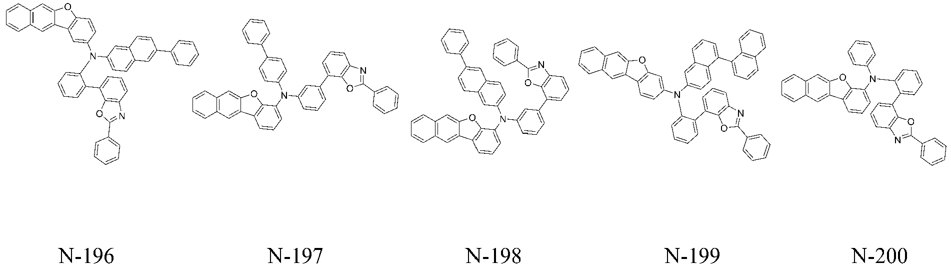

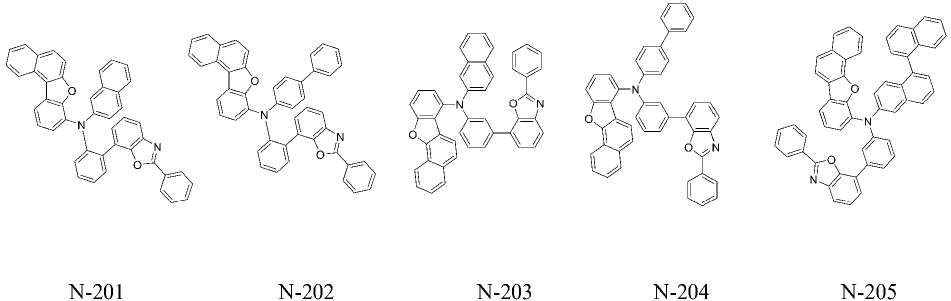

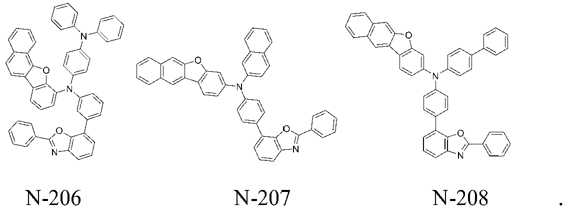

The present application provides a luminescent host material comprising the benzonaphthofuran-containing oxazole organic compound as described above.

Optionally, the luminescent host material comprises a first host material and a second host material, wherein the first host material is the benzonaphthofuran-containing oxazole organic compound as described above; and the second host material is an organic electroluminescent compound having a structure represented by Formula (2):

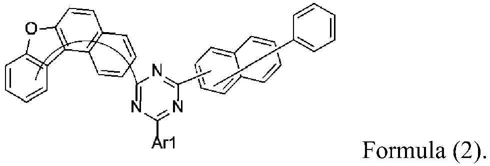

In Formula (2), Ar1 is selected from the group consisting of substituted or unsubstituted C6-C60 aryl, and substituted or unsubstituted C3-C60 heteroaryl; and
the substituents in the substituted C6-C60 aryl and the substituted C3-C60 heteroaryl are each independently selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof.

Optionally, Ar1 is selected from the group consisting of substituted or unsubstituted C6-C60 non-fused aryl, and substituted or unsubstituted C3-C60 non-fused heteroaryl;
the substituents in the substituted C6-C60 non-fused aryl and the substituted C3-C60 non-fused heteroaryl are each independently selected from the group consisting of deuterium, halogen, cyano, C1-C12 alkyl, C3-C12 cycloalkyl, C6-C30 aryl, C3-30 heteroaryl, C6-C60 arylamine, and C3-C60 heteroarylamine, or a combination of at least two thereof.

Optionally, Ar1 is selected from the group consisting of substituted or unsubstituted C6-C20 non-fused aryl, and substituted or unsubstituted C3-C20 non-fused heteroaryl;
the substituents in the substituted C6-C20 non-fused aryl and substituted C3-C20 non-fused heteroaryl are each independently selected from the group consisting of deuterium, halogen, cyano, C1-C12 alkyl, C3-C12 cycloalkyl, C6-C30 aryl, C3-C30 heteroaryl, C6-C60 arylamine, and C3-C60 heteroarylamine, or a combination of at least two thereof.

Optionally, Ar1 is selected from substituted or unsubstituted A group, and the A group is selected from the group consisting of phenyl, biphenyl, and triphenyl;
wherein the substituent in the substituted A is selected from the group consisting of deuterium, phenyl and naphthyl; and
optionally, Ar1 is selected from the group consisting of phenyl, biphenyl, triphenyl and naphthylphenyl.

Optionally, the organic electroluminescent compound having a structure represented by Formula (2) is selected from any one of M-1 to M-104 as follows:

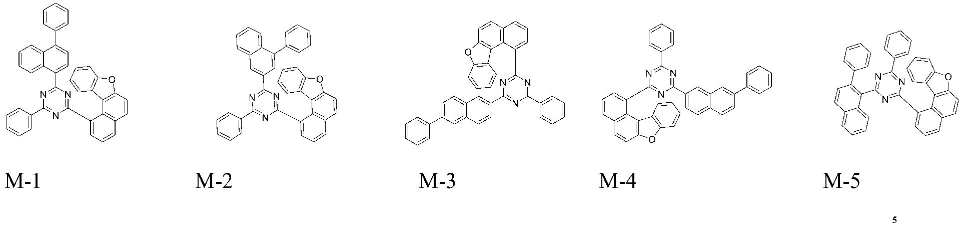

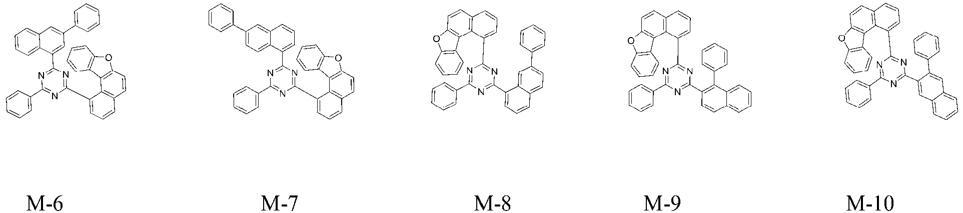

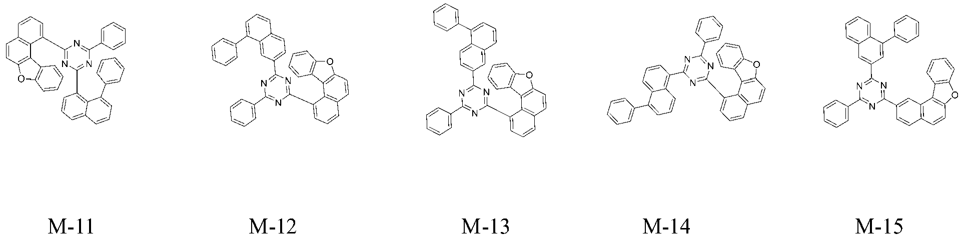

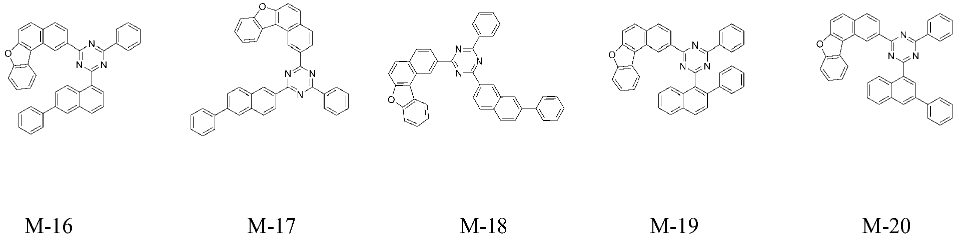

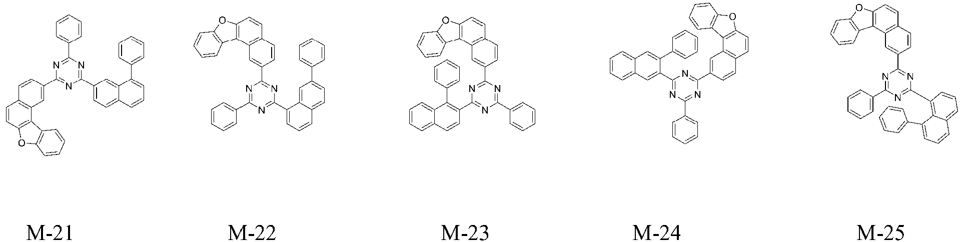

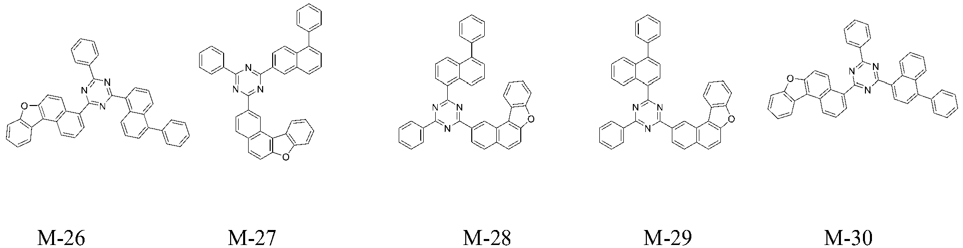

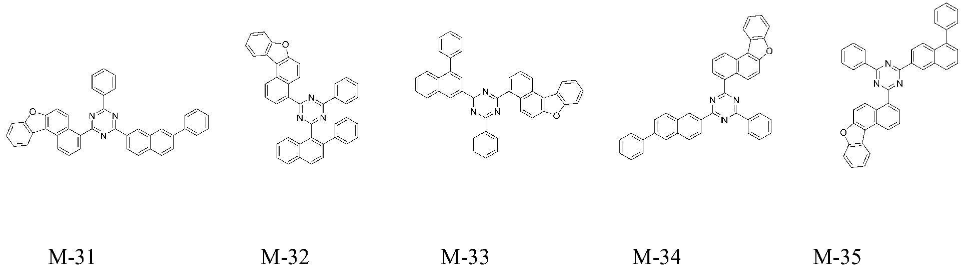

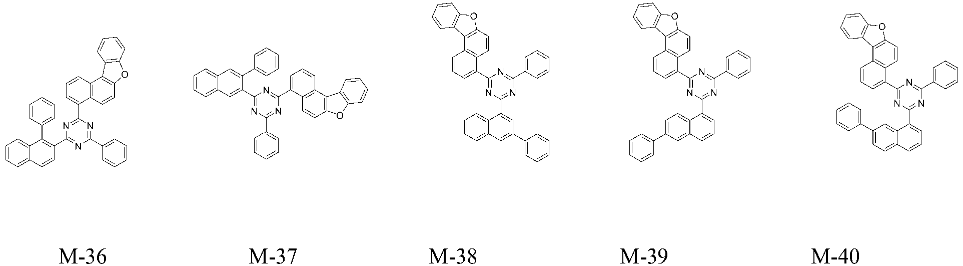

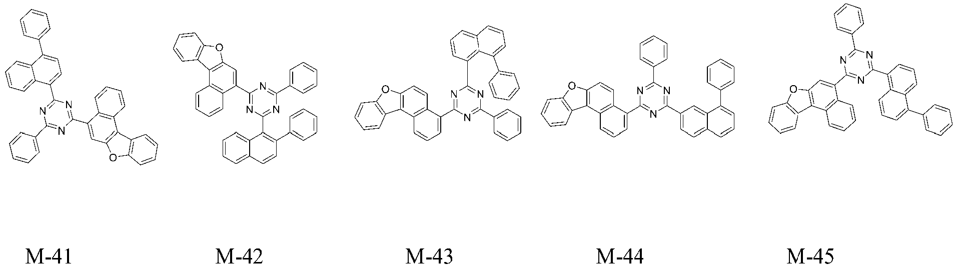

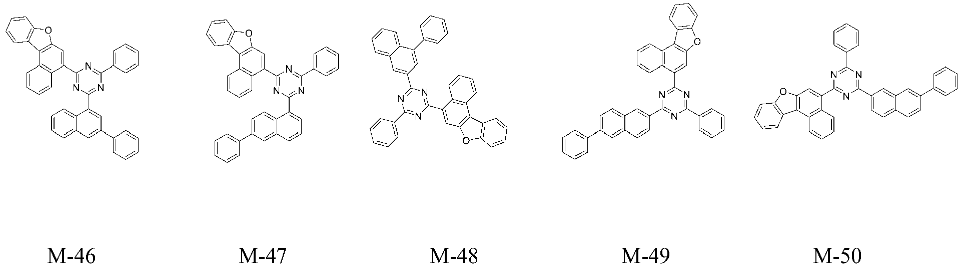

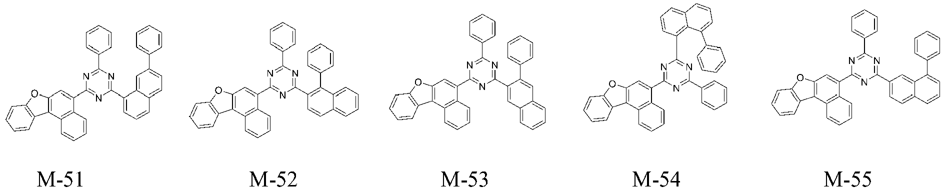

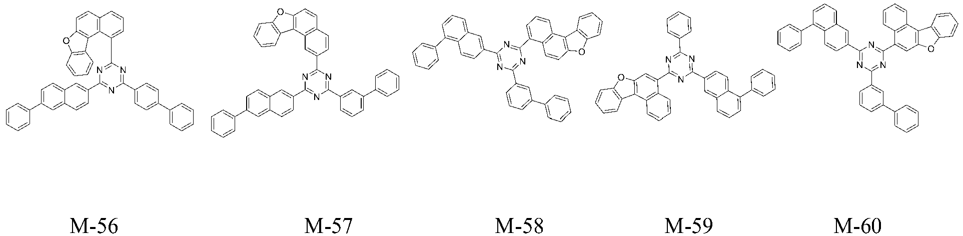

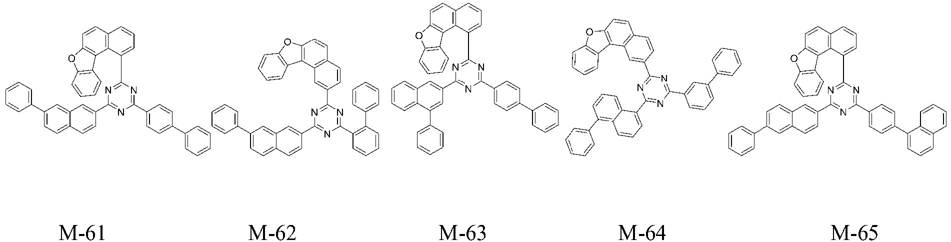

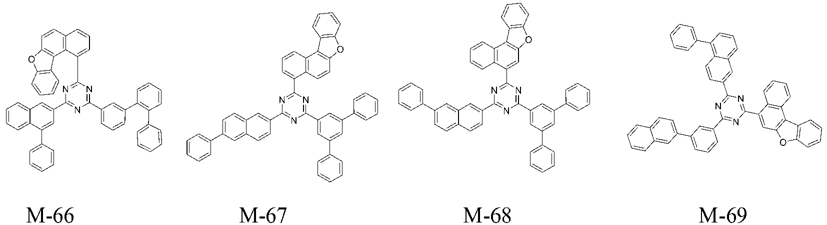

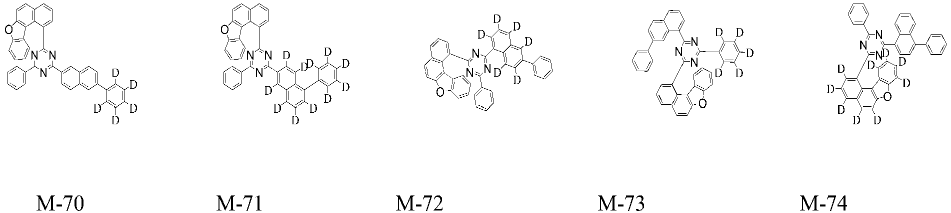

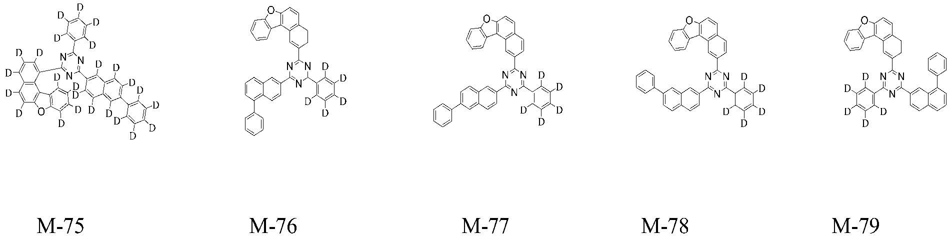

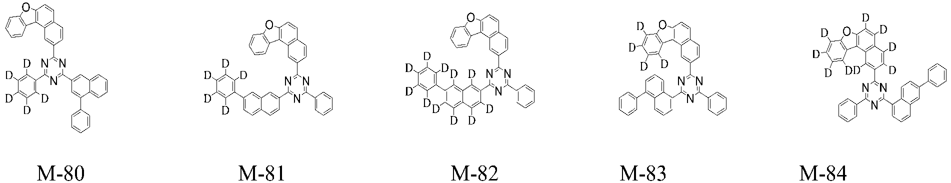

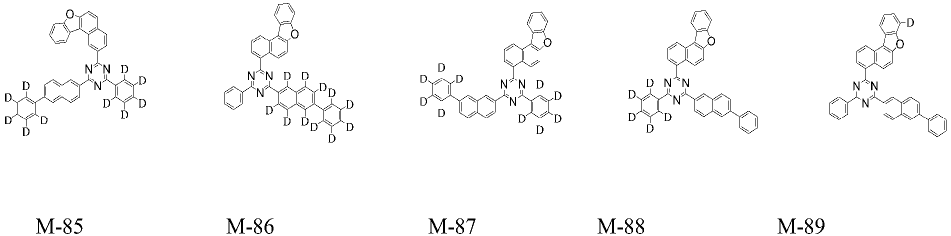

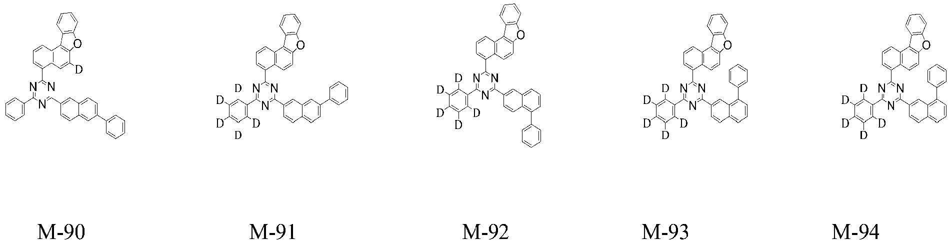

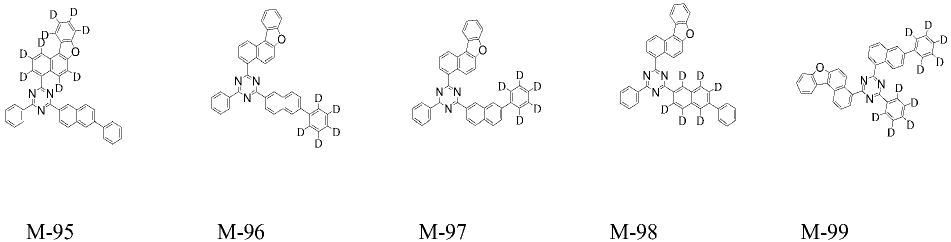

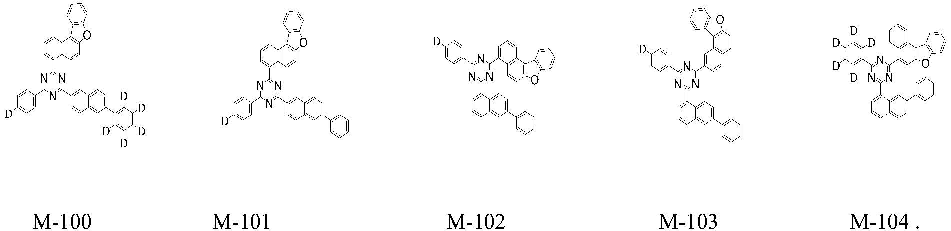

Optionally, a mass ratio of the first host material to the second host material is 9:1-1:9;
optionally, a mass ratio of the first host material to the second host material is 2:8-8:2;
more optionally, a mass ratio of the first host material to the second host material is 3:7-7:3;
further optionally, a mass ratio of the first host material to the second host material is 4:6-6:4.

The present application also provides an organic electroluminescent material, comprising the above-mentioned benzonaphthofuran-containing oxazole organic compound, or the above-mentioned luminescent host material.

The present application provides an organic electroluminescent device comprising a cathode, an anode, and an organic layer disposed between the cathode and the anode, wherein the organic layer comprises the above-mentioned benzonaphthofuran-containing oxazole organic compound, the above-mentioned luminescent host material, or the above-mentioned organic electroluminescent material;
optionally, the organic layer comprises one or more of a hole injection layer, a hole transport layer, a light-emitting layer, an electron transport layer, and an electron injection layer,
optionally, the hole transport layer comprises the above-mentioned benzonaphthofuran-containing oxazole organic compound, the above-mentioned luminescent host material, or the above-mentioned organic electroluminescent material; and
optionally, the light-emitting layer comprises the above-mentioned benzonaphthofuran-containing oxazole organic compound, the above-mentioned luminescent host material, or the above-mentioned organic electroluminescent material.

The present application provides the use of the above-mentioned organic electroluminescent device in fiber optic equipment, lighting equipment, electronic photographic photosensitive equipment, photoelectric converter, organic solar cell, switching element equipment, organic light-emitting field-effect transistor, image sensor, or dye laser.

The beneficial effects of the present application are as follows.

The present application provides a benzonaphthofuran-containing oxazole organic compound based on the structure of Formula (1), in which the types of substituents are further defined, which can improve the structural stability of the compound. Moreover, the HOMO and LUMO energy levels of the benzonaphthofuran-containing oxazole organic compound have a high degree of matching with adjacent energy levels, resulting in a relatively balanced carrier mobility of the benzonaphthofuran-containing oxazole organic compound, thereby enabling organic electroluminescent devices containing this benzonaphthofuran-containing oxazole organic compound to have relatively low driving voltage, relatively high luminous efficiency, and relatively long lifetime.

Furthermore, the benzonaphthofuran-containing oxazole organic compound provided in the present application have relatively good electron transfer properties and can be used as electron transfer materials or luminescent materials.

Furthermore, the present application provides an organic electroluminescent material comprising a benzonaphthofuran-containing oxazole organic compound based on the structure of Formula (1), thereby enabling organic electroluminescent devices containing the organic electroluminescent material to have relatively low driving voltage, relatively high luminous efficiency, and relatively long lifespan.

### BRIEF DESCRIPTION OF THE DRAWINGS

In order to more clearly illustrate the specific implementation methods of the present application or the technical solutions in the prior art, the drawings required for use in the specific implementation methods or the description of the prior art will be briefly introduced below. Obviously, the drawings described below are some implementation methods of the present application. For those skilled in the art, other drawings can be obtained based on these drawings without paying creative work.

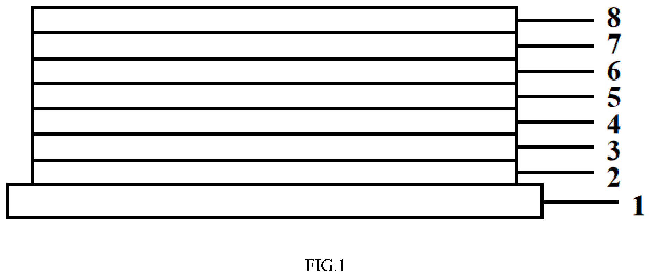

FIG. 1 is a structural diagram of an organic electroluminescent device in an embodiment of the device according to the present application;
wherein the reference signs are as follows: 1-substrate; 2-anode; 3-hole injection layer; 4-hole transport layer; 5-light-emitting layer; 6-electron transport layer; 7-electron injection layer; 8-cathode.

### DETAILED DESCRIPTION

The following examples are provided for a better understanding of the present application, but are not intended to limit the best implementation, nor to limit the content and protection scope of the present application. Any product identical or similar to the present application obtained by anyone under the inspiration of the present application or by combining the features of the present application with other prior arts shall fall within the protection scope of the present application.

If no specific experimental steps or conditions are specified in the examples, the experiments can be carried out according to the conventional experimental steps or conditions described in the literature in the art. The reagents or instruments used without indicating the manufacturer are all conventional reagent products that can be obtained through commercial purchase.

The term "organic electroluminescent material" in this disclosure means a material that can be used in organic electroluminescent devices and may include at least one compound. If necessary, the organic electroluminescent material may be contained in any layer constituting the organic electroluminescent device. For example, the organic electroluminescent material can be a hole injection material, a hole transport material, a hole-assisting material, an emission-assisting material, an electron blocking material, a luminescent material (containing an organic electroluminescent main material and a dopant material), an electron buffer material, a hole blocking material, an electron transport material, an electron injection material, and so on.

The term "plurality of organic electroluminescent materials" in this disclosure refers to one or more organic electroluminescent materials comprising a combination of at least two compounds, and the materials may be included in any layer constituting an organic electroluminescent device. It may mean both a material included before the organic electroluminescent devices (e.g. before vapor deposition) and materials included after (for example, after vapor deposition) and a material included after the organic electroluminescent device (for example, after vapor deposition). For example, plurality of organic electroluminescent materials may be a combination of at least two compounds, which can be included in at least one of the following: a hole injection layer, a hole transport layer, a hole-assisting layer, an emission-assisting layer, an electron blocking layer, a light-emitting layer, an electron buffer layer, a hole blocking layer, an electron transport layer and an electron injection layer. At least two compounds can be contained in the same layer or in different layers, and may be mix-evaporated or co-evaporated, or may be evaporated individually.

In the present application, the benzonaphthofuran-containing oxazole organic compound having a structure of Formula (1) can be prepared by the following synthetic route:
1. Synthesis of intermediate Nn-A: the reaction raw materials Nn-1 and Nn-2 are subjected to Suzuki cross-coupling reaction, and the reaction equation is as follows: 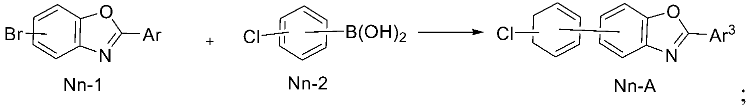
2. Synthesis of compound N-n: intermediate Nn-A and Nn-B undergo Buchwald-Hartwig cross-coupling reaction, the reaction equation is as follows:

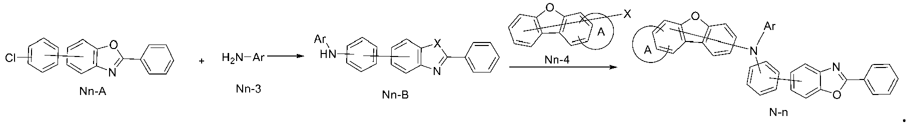

In the present application, the compound having the structure represented by Formula (2) is prepared by the following synthetic route:

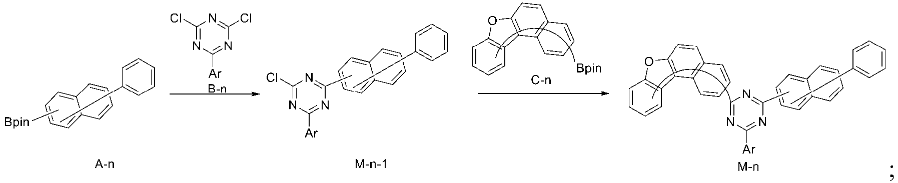

Intermediate raw materials A-n, B-n, and C-n can be directly purchased or synthesized through conventional reaction routes and conditions by referring to existing literature reports.

The specific structure of the intermediate raw materials used in the examples provided in the present application is as follows:
The specific structure of B-n is as follows:

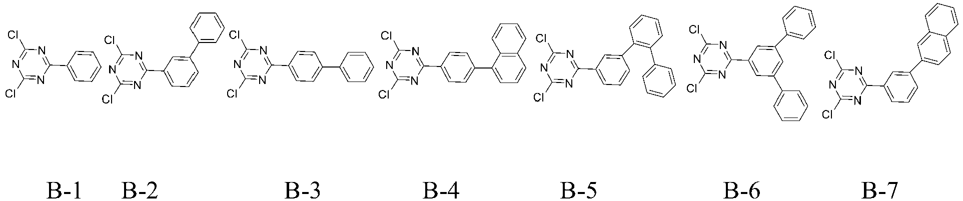

The specific structure of C-n is as follows:

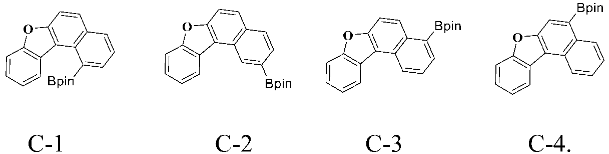

### Example 1

This example provides a benzonaphthofuran-containing oxazole organic compound **N-1.** The synthesis of the benzonaphthofuran-containing oxazole organic compound N-1 specifically comprises the following steps:

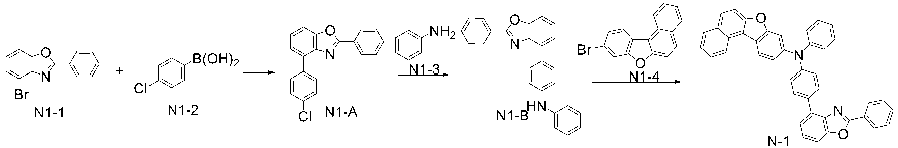

### Synthesis of intermediate N1-A

After replacing the air in a three port reaction bottle equipped with mechanical stirring, thermometer, and condenser tube with nitrogen, the raw materials 10 g of N1-1 (36.63 mmol), 5.72 g of N1-2 (36.63 mmol), 0.85 g of tetrakistriphenylphosphine palladium (0.73 mmol), 10.11 g of potassium carbonate (73.27 mmol), 70 mL of toluene, 30 mL of ethanol and 30 mL of water were added in sequence. The mixture was stirred at 65°C for 2 hours. After the reaction was completed, the organic layer was extracted with ethyl acetate (EA), and residual moisture was removed by using anhydrous sodium sulfate, and the residue was dried and purified by column chromatography to obtain 10.06 g of compound N1-A (yield 90%).

### Synthesis of intermediate N1-B

After replacing the air in a three port reaction bottle equipped with mechanical stirring, thermometer, and condenser tube with nitrogen, 10 g of the intermediate N1-A (32.78 mmol), 3.1 g of N1-3 (32.78 mmol), 0.6g of tris(dibenzylideneacetone)dipalladium (0.66 mmol), 0.67 g of 2-dicyclohexylphosphine-2',6'-dimethoxybiphenyl (1.64 mmol), 6.3 g of sodium tert-butoxide (65.56 mmol) and 100mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 8.31 g of compound N1-B (yield 82%).

### Synthesis of Compound N-1

After replacing the air in a three port reaction bottle equipped with mechanical stirring, thermometer, and condenser tube with nitrogen, 8.30 g of the intermediate N1-B (26.85 mmol), 7.95 g of N1-4 (26.85 mmol), 0.49 g of tris(dibenzylideneacetone)dipalladium (0.54 mmol), 0.55 g of 2-dicyclohexylphosphine-2',6'-dimethoxybiphenyl (1.34 mmol), 5.16 g of sodium tert-butoxide (53.70 mmol) and 100mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 11.80 g of benzonaphthofuran-containing oxazole organic compound N-1 (yield 76%).

Elemental analysis: C₄₁H₂₆N₂O₂; theoretical value: C, 85.10; H, 4.53; N, 4.84; O, 5.53; measured value: C, 85.12; H, 4.52; N, 4.83; HRMS(ESI)m/z [M+H]+: theoretical value: 578.20; measured value: 579.21.

### Example 2

This example provides a benzonaphthofuran-containing oxazole organic compound **N-6.** The synthesis of the benzonaphthofuran-containing oxazole organic compound **N-6** specifically comprises the following steps:

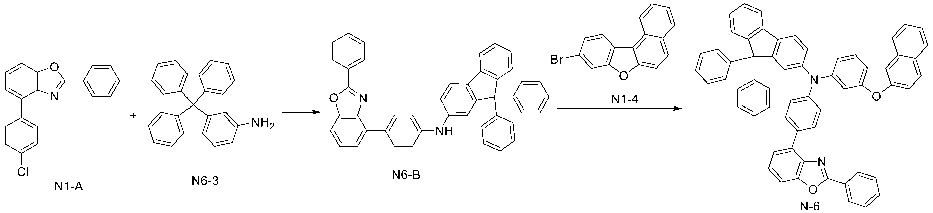

After replacing the air in a three port reaction bottle equipped with mechanical stirring, thermometer, and condenser tube with nitrogen, 10 g of the intermediate N1-A (32.78 mmol), 10.92 g of N6-3 (32.78 mmol), 0.6 g of tris(dibenzylideneacetone)dipalladium (0.66 mmol), 0.67 g of 2-dicyclohexylphosphine-2',6'-dimethoxybiphenyl (1.64 mmol), 6.3 g of sodium tert-butoxide (65.56 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 14.41 g of compound N6-B (yield 73%).

After replacing the air in a three port reaction bottle equipped with mechanical stirring, thermometer, and condenser tube with nitrogen, 10 g of the intermediate N6-B (16.60 mmol), 4.91 g of N1-4 (16.60 mmol), 0.30 g of tris(dibenzylideneacetone)dipalladium (0.33 mmol), 0.34 g of 2-dicyclohexylphosphine-2',6'-dimethoxybiphenyl (0.83 mmol), 3.19 g of sodium tert-butoxide (33.21 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 9.24 g of benzonaphthofuran-containing oxazole organic compound N-6 (yield 68%).

Elemental analysis: C₆₀H₃₈N₂O₂; theoretical value: C, 88.00; H, 4.68; N, 3.42; O, 3.91; measured value: C, 88.01; H, 4.68; N, 3.41; HRMS(ESI)m/z [M+H]+: theoretical value: 818.29; measured value: 819.28.

### Example 3

This example provides a benzonaphthofuran-containing oxazole organic compound **N-18.** The synthesis of the benzonaphthofuran-containing oxazole organic compound **N-18** specifically comprises the following steps:

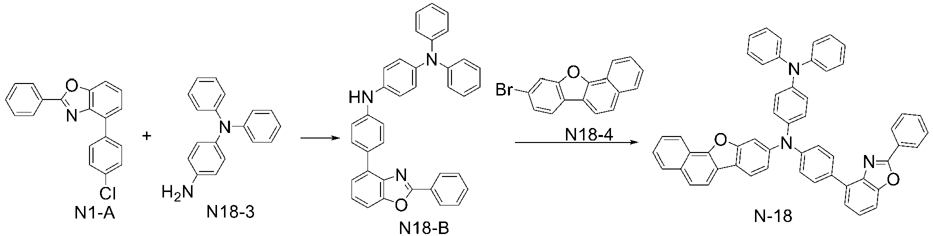

After replacing the air in a three port reaction bottle equipped with mechanical stirring, thermometer, and condenser tube with nitrogen, 10 g of the intermediate N1-A (32.78 mmol), 8.53 g of N18-3 (32.78 mmol), 0.60 g of tris(dibenzylideneacetone)dipalladium (0.66 mmol), 0.67 g of 2-dicyclohexylphosphine-2',6'-dimethoxybiphenyl (1.64 mmol), 6.3 g of sodium tert-butoxide (65.56 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 13.19 g of compound N18-B (yield 76%).

After replacing the air in a three port reaction bottle equipped with mechanical stirring, thermometer, and condenser tube with nitrogen, 10 g of N18-B (18.90 mmol), 5.59 g of N18-4 (18.90 mmol), 0.35 g of tris(dibenzylideneacetone)dipalladium (0.38 mmol), 0.39 g of 2-dicyclohexylphosphine-2',6'-dimethoxybiphenyl (0.94 mmol), 3.63 g of sodium tert-butoxide (37.79 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.70 g of organic electroluminescent compound N-18 (yield 76%).

Elemental analysis: C₅₃H₃₅N₃O₂; theoretical value: C, 85.35; H, 4.73; N, 5.63; O, 4.29; measured value: C, 85.37; H, 4.72; N, 5.62; HRMS(ESI)m/z [M+H]+: theoretical value: 745.27; measured value: 746.26.

### Example 4

This example provides a benzonaphthofuran-containing oxazole organic compound **N-30.** The synthesis of the benzonaphthofuran-containing oxazole organic compound **N-30** specifically comprises the following steps:

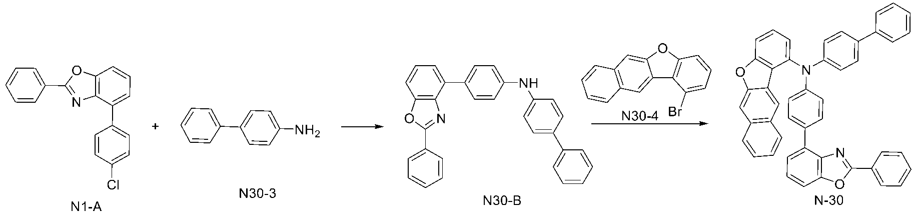

After replacing the air in a three port reaction bottle equipped with mechanical stirring, thermometer, and condenser tube with nitrogen, 10 g of the intermediate N1-A (32.78 mmol), 5.54 g of N30-3 (32.78 mmol), 0.60 g of tris(dibenzylideneacetone)dipalladium (0.66 mmol), 0.67 g of 2-dicyclohexylphosphine-2',6'-dimethoxybiphenyl (1.64 mmol), 6.30 g of sodium tert-butoxide (65.56 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 11.49 g of compound N30-B (yield 80%).

After replacing the air in a three port reaction bottle equipped with mechanical stirring, thermometer, and condenser tube with nitrogen, 10 g of the intermediate N30-B (22.82 mmol), 6.75 g of N30-4 (22.82 mmol), 0.42 g of tris(dibenzylideneacetone)dipalladium (0.46 mmol), 0.47 g of 2-dicyclohexylphosphine-2',6'-dimethoxybiphenyl (1.14 mmol), 4.39 g of sodium tert-butoxide (45.64 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.75 g of benzonaphthofuran-containing oxazole organic compound N-30 (yield 72%).

Elemental analysis: C₄₇H₃₀N₂O₂; theoretical value: C, 86.22; H, 4.62; N, 4.28; O, 4.89; measured value: C, 86.20; H, 4.63; N, 4.29; HRMS(ESI)m/z [M+H]+: theoretical value: 654.23; measured value: 654.24.

### Example 5

This example provides a benzonaphthofuran-containing oxazole organic compound **N-45.** The synthesis of the benzonaphthofuran-containing oxazole organic compound **N-45** specifically comprises the following steps:

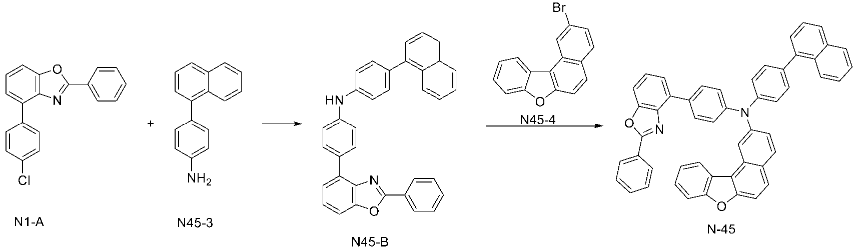

After replacing the air in a three port reaction bottle equipped with mechanical stirring, thermometer, and condenser tube with nitrogen, 10 g of the intermediate N1-A (32.78 mmol), 7.18 g of N45-3 (32.78 mmol), 0.60 g of tris(dibenzylideneacetone)dipalladium (0.66 mmol), 0.67 g of 2-dicyclohexylphosphine-2',6'-dimethoxybiphenyl (1.64 mmol), 6.30 g of sodium tert-butoxide (65.56 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 12.16 g of compound N45-B (yield 76%).

After replacing the air in a three port reaction bottle equipped with mechanical stirring, thermometer, and condenser tube with nitrogen, 7.50 g of the intermediate N45-B (15.36 mmol), 3.87 g of N45-4 (15.36 mmol), 0.28 g of tris(dibenzylideneacetone)dipalladium (0.31 mmol), 0.31 g of 2-dicyclohexylphosphine-2',6'-dimethoxybiphenyl (0.77 mmol), 2.95 g of sodium tert-butoxide (30.73 mmol) and 80 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 8.22 g of benzonaphthofuran-containing oxazole organic compound N-45 (yield 76%).

Elemental analysis: C₅₁H₃₂N₂O₂; theoretical value: C, 86.91; H, 4.58; N, 3.97; O, 4.54; measured value: C, 86.93; H, 4.57; N, 3.96; HRMS(ESI)m/z [M+H]+: theoretical value: 704.25; measured value: 705.26.

### Example 6

This example provides a benzonaphthofuran-containing oxazole organic compound **N-72.** The synthesis of the benzonaphthofuran-containing oxazole organic compound **N-72** specifically comprises the following steps:

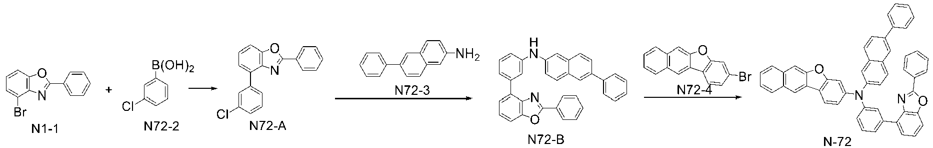

### Synthesis of intermediate N72-A

After replacing the air in a three port reaction bottle equipped with mechanical stirring, thermometer, and condenser tube with nitrogen, the raw materials 10 g of N1-1 (36.63 mmol), 5.72 g of N72-2 (36.63 mmol), 0.85 g of tetrakistriphenylphosphine palladium (0.73 mmol), 10.11 g of potassium carbonate (73.27 mmol), 70 mL of toluene, 30 mL of ethanol and 30 mL of water were added in sequence. The mixture was stirred at 65°C for 2 hours. After the reaction was completed, the organic layer was extracted with ethyl acetate (EA), and residual moisture was removed by using anhydrous sodium sulfate, the residue was dried and purified by column chromatography to obtain 9.50 g of compound N72-A (yield 85%).

### Synthesis of intermediate N72-B

After replacing the air in a three port reaction bottle equipped with mechanical stirring, thermometer, and condenser tube with nitrogen, 9.50 g of the intermediate N72-A (31.14 mmol), 6.82 g of N73-3 (31.14 mmol), 0.57 g of tris(dibenzylideneacetone)dipalladium (0.62 mmol), 0.64 g of 2-dicyclohexylphosphine-2',6'-dimethoxybiphenyl (1.56 mmol), 5.99 g of sodium tert-butoxide (62.28 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 11.10 g of compound N72-B (yield 73%).

After replacing the air in a three port reaction bottle equipped with mechanical stirring, thermometer, and condenser tube with nitrogen, 10 g of the intermediate N72-B (20.48 mmol), 6.06 g of N72-4 (20.48 mmol), 0.37 g of tris(dibenzylideneacetone)dipalladium (0.41 mmol), 0.42 g of 2-dicyclohexylphosphine-2',6'-dimethoxybiphenyl (1.02 mmol), 3.94 g of sodium tert-butoxide (40.97 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.82 g of benzonaphthofuran-containing oxazole organic compound N-72 (yield 75%).

Elemental analysis: C₅₁H₃₂N₂O₂; theoretical value: C, 86.91; H, 4.58; N, 3.97; O, 4.54; measured value: C, 86.93; H, 4.57; N, 3.96;HRMS(ESI)m/z [M+H]+: theoretical value: 704.25; measured value: 705.26.

### Example 7

This example provides a benzonaphthofuran-containing oxazole organic compound **N-95.** The synthesis of the benzonaphthofuran-containing oxazole organic compound **N-95** specifically comprises the following steps:

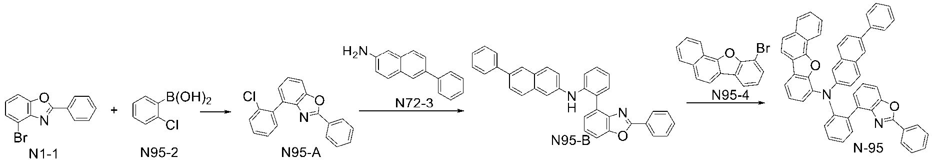

After replacing the air in a three port reaction bottle equipped with mechanical stirring, thermometer, and condenser tube with nitrogen, the raw materials 10 g of N1-1 (36.63 mmol), 5.72 g of N95-2 (36.63mmol), 0.85 g of tetrakistriphenylphosphine palladium (0.73 mmol), 10.11g of potassium carbonate (73.27 mmol), 70 mL of toluene, 30 mL of ethanol and 30 mL of water were added in sequence. The mixture was stirred at 65°C for 2 hours. After the reaction was completed, the organic layer was extracted with ethyl acetate (EA), and residual moisture was removed by using anhydrous sodium sulfate, and the residue was dried and purified by column chromatography to obtain 9.28 g of compound N95-A (yield 83%).

After replacing the air in a three port reaction bottle equipped with mechanical stirring, thermometer, and condenser tube with nitrogen, 9.28 g of the intermediate N95-A (30.42 mmol), 6.67 g of N72-3 (30.42 mmol), 0.56 g of tris(dibenzylideneacetone)dipalladium (0.61 mmol), 0.62 g of 2-dicyclohexylphosphine-2',6'-dimethoxybiphenyl (1.52 mmol), 5.85 g of sodium tert-butoxide (60.84 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 10.69 g of compound N95-B (yield 72%).

After replacing the air in a three port reaction bottle equipped with mechanical stirring, thermometer, and condenser tube with nitrogen, 10 g of the intermediate N95-B (20.48 mmol), 6.06 g of N95-4 (20.48 mmol), 0.37 g of tris(dibenzylideneacetone)dipalladium (0.41 mmol), 0.42 g of 2-dicyclohexylphosphine-2',6'-dimethoxybiphenyl (1.02 mmol), 3.94 g of sodium tert-butoxide (40.97 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.10 g of benzonaphthofuran-containing oxazole organic compound N-95 (yield 70%).

Elemental analysis: C₅₁H₃₂N₂O₂; theoretical value: C, 86.91; H, 4.58; N, 3.97; O, 4.54; measured value: C, 86.90; H, 4.58; N, 3.98; HRMS(ESI)m/z [M+H]+: theoretical value: 704.25; measured value: 705.26.

### Example 8

This example provides a benzonaphthofuran-containing oxazole organic compound **N-134.** The synthesis of the benzonaphthofuran-containing oxazole organic compound **N-134** specifically comprises the following steps:

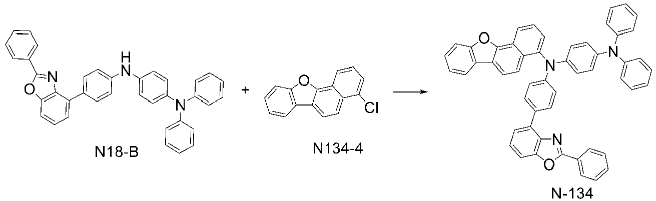

After replacing the air in a three port reaction bottle equipped with mechanical stirring, thermometer, and condenser tube with nitrogen, 8.60 g of the intermediate N18-B (16.25 mmol), 4.10 g of N134-4 (16.25 mmol), 0.30 g of tris(dibenzylideneacetone)dipalladium (0.33 mmol), 0.33 g of 2-dicyclohexylphosphine-2',6'-dimethoxybiphenyl (0.81 mmol), 3.12 g of sodium tert-butoxide (32.50 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 9.20 g of benzonaphthofuran-containing oxazole organic compound N-134 (yield 76%).

Elemental analysis: C₅₃H₃₅N₃O₂; theoretical value: C, 85.35; H, 4.73; N, 5.63; O, 4.29; measured value: C, 85.33; H, 4.74; N, 5.64;HRMS(ESI)m/z [M+H]+: theoretical value: 745.27; measured value: 746.29.

### Example 9

This example provides a benzonaphthofuran-containing oxazole organic compound **N-148.** The synthesis of the benzonaphthofuran-containing oxazole organic compound **N-148** specifically comprises the following steps:

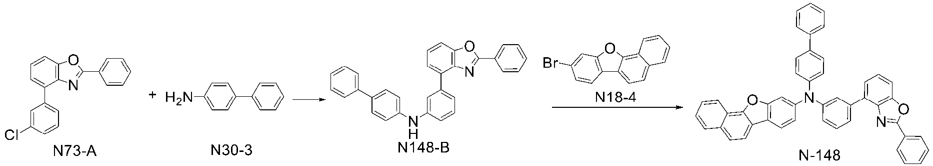

After replacing the air in a three port reaction bottle equipped with mechanical stirring, thermometer, and condenser tube with nitrogen, the intermediate 10 g of N73-A (32.78 mmol), 5.54 g of N30-3 (32.78 mmol), 0.60 g of tris(dibenzylideneacetone)dipalladium (0.66 mmol), 0.67 g of 2-dicyclohexylphosphine-2',6'-dimethoxybiphenyl (1.64mmol), 6.3 g of sodium tert-butoxide (65.56mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 11.34 g of compound N148-B (yield 79%).

After replacing the air in a three port reaction bottle equipped with mechanical stirring, thermometer, and condenser tube with nitrogen, 10 g of the intermediate N148-B (22.82 mmol), 6.57 g of N18-4 (22.82 mmol), 0.42 g of tris(dibenzylideneacetone)dipalladium (0.46 mmol), 0.47 g of 2-dicyclohexylphosphine-2',6'-dimethoxybiphenyl (1.14 mmol), 4.39 g of sodium tert-butoxide (45.64 mmol) and 100 mL of toluene were added in sequence, and the mixture was refluxed and stirred at 110° C. for 3 hours. After the reaction was completed, the organic layer was extracted with ethyl acetate (EA), and residual moisture was removed by using anhydrous sodium sulfate, the residue was dried and purified by column chromatography to obtain 11.20 g of benzonaphthofuran-containing oxazole organic compound N-148 (yield 75%).

Elemental analysis: C₄₇H₃₀N₂O₂; theoretical value: C, 86.22; H, 4.62; N, 4.28; O, 4.89; measured value: C, 86.22; H, 4.62; N, 4.28;HRMS(ESI)m/z [M+ H]+: theoretical value: 654.23; measured value: 655.21.

### Example 10

This example provides an organic electroluminescent compound N-173. The synthesis of the organic electroluminescent compound N-173 specifically comprises the following steps:

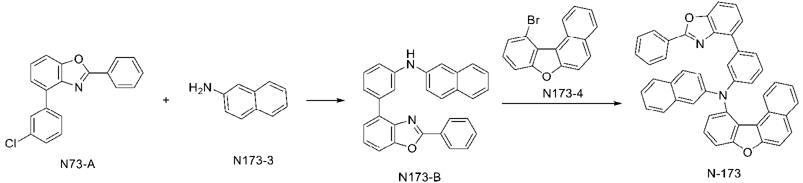

After replacing the air in a three port reaction bottle equipped with mechanical stirring, thermometer, and condenser tube with nitrogen, the intermediate 10 g of N73-A (32.78 mmol), 4.69 g of N173-3 (32.78 mmol), 0.60 g of tris(dibenzylideneacetone)dipalladium (0.66 mmol), 0.67 g of 2-dicyclohexylphosphine-2',6'-dimethoxybiphenyl (1.64 mmol), 6.30 g of sodium tert-butoxide (65.56 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, and the residue was dried and purified by column chromatography to obtain 9.73 g of compound N173-B (yield 72%).

After replacing the air in a three port reaction bottle equipped with mechanical stirring, thermometer, and condenser tube with nitrogen, 9.70 g of the intermediate N173-B (23.53 mmol), 6.97 g of N173-4 (23.53 mmol), 0.43 g of tris(dibenzylideneacetone)dipalladium (0.47 mmol), 0.48 g of 2-dicyclohexylphosphine-2',6'-dimethoxybiphenyl (1.18 mmol), 4.52 g of sodium tert-butoxide (47.07 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.35 g of compound N-173 (yield 70%).

Elemental analysis: C₄₅H₂₈N₂O₂; theoretical value: C, 85.97; H, 4.49; N, 4.46; O, 5.09; measured value: C, 85.96; H, 4.48; N, 4.48;HRMS(ESI)m/z [M+H]+: theoretical value: 628.22; measured value: 629.24.

### Example 11

This example provides an organic electroluminescent compound N-182. The synthesis of the organic electroluminescent compound N-182 specifically comprises the following steps:

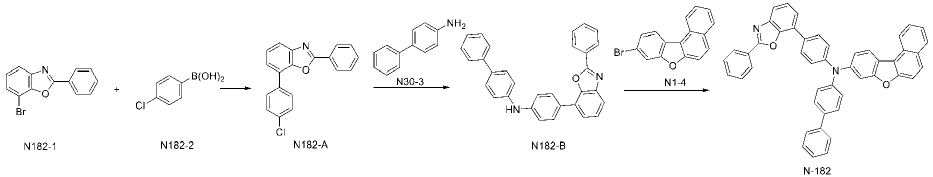

After replacing the air in a three port reaction bottle equipped with mechanical stirring, thermometer, and condenser tube with nitrogen, the raw materials 10 g of N182-1 (36.63 mmol), 5.72 g of N182-2 (36.63 mmol), 0.85g of tetrakistriphenylphosphine palladium (0.73 mmol), 10.11g of potassium carbonate (73.27 mmol), 70 mL of toluene, 30 mL of ethanol and 30 mL of water were added in sequence. The mixture was stirred at 65°C for 2 hours. After the reaction was completed, the organic layer was extracted with ethyl acetate (EA), and residual moisture was removed by using anhydrous sodium sulfate, and the residue was dried and purified by column chromatography to obtain 8.94 g of compound N182-A (yield 80%).

After replacing the air in a three port reaction bottle equipped with mechanical stirring, thermometer, and condenser tube with nitrogen, 8.94 g of the intermediate N182-A (29.31 mmol), 4.96 g of N30-3 (29.31 mmol), 0.54 g of tris(dibenzylideneacetone) dipalladium (0.59 mmol), 0.60 g of 2-dicyclohexylphosphine-2',6'-dimethoxybiphenyl (1.47 mmol), 5.63 g of sodium tert-butoxide (58.61 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.01 g of compound N182-B (yield 78%).

After replacing the air in a three port reaction bottle equipped with mechanical stirring, thermometer, and condenser tube with nitrogen, 10.01 g of the intermediate N182-B (22.85 mmol), 6.76 g of N1-4 (22.85 mmol), 0.42 g of tris(dibenzylideneacetone) dipalladium (0.46 mmol), 0.47 g of 2-dicyclohexylphosphine-2',6'-dimethoxybiphenyl (1.14 mmol), 4.39 g of sodium tert-butoxide (45.69 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 11.21 g of compound N-182 (yield 75%).

Elemental analysis: C₄₇H₃₀N₂O₂; theoretical value: C, 86.22; H, 4.62; N, 4.28; O, 4.89; measured value: C, 86.24; H, 4.61; N, 4.27;HRMS(ESI)m/z [M+H]+: theoretical value: 654.23; measured value: 654.21.

### Example 12

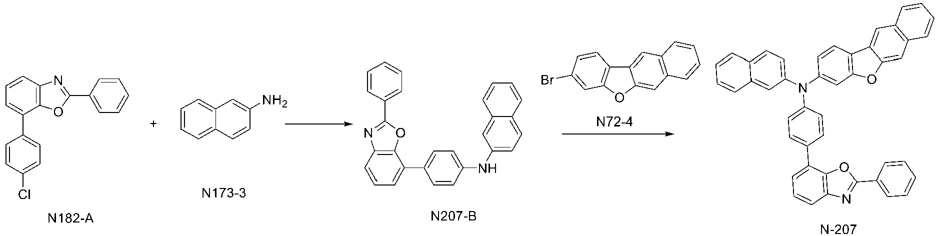

After replacing the air in a three port reaction bottle equipped with mechanical stirring, thermometer, and condenser tube with nitrogen, 8.94 g of the intermediate N182-A (29.31 mmol), 4.96 g of N173-3 (29.31 mmol), 0.54 g of tris(dibenzylideneacetone) dipalladium (0.59 mmol), 0.60 g of 2-dicyclohexylphosphine-2',6'-dimethoxybiphenyl (1.47 mmol), 5.63 g of sodium tert-butoxide (58.61 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.01 g of compound N207-B (yield 78%).

After replacing the air in a three port reaction bottle equipped with mechanical stirring, thermometer, and condenser tube with nitrogen, 10 g of the intermediate N207-B (24.26 mmol), 7.18 g of N72-4 (24.26 mmol), 0.44 g of tris(dibenzylideneacetone)dipalladium (0.49 mmol), 0.50 g of 2-dicyclohexylphosphine-2',6'-dimethoxybiphenyl (1.21 mmol), 4.66 g of sodium tert-butoxide (48.52 mmol) and 100 mL of toluene were added in sequence, and the mixture was refluxed and stirred at 110°C for 3 hours. After the reaction was completed, the organic layer was extracted with ethyl acetate (EA), and residual moisture was removed by using anhydrous sodium sulfate, the residue was dried and purified by column chromatography to obtain 10.97 g of compound N-207 (yield 72%).

Elemental analysis: C₄₅H₂₈N₂O₂; theoretical value: C, 85.97; H, 4.49; N, 4.46; O, 5.09; measured value: C, 85.95; H, 4.49; N, 4.48; HRMS(ESI)m/z [M+H]+: theoretical value: 628.22; measured value: 628.21.

The preparation methods of Examples 13-21 are similar to those of Example 1. Specifically, the raw materials used in Examples 13-21 and the products obtained are shown in Table 1 below.

**Table 1**

| Example | Intermediate Nn-B | Raw material Nn-4 | Product | Yield% |
|---|---|---|---|---|
| 13 | 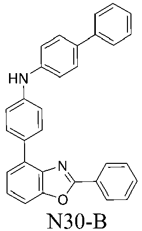 | 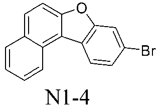 | 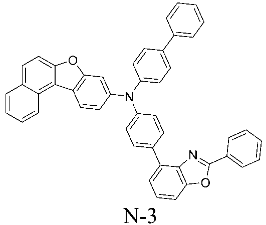 | 78% |
| 14 | 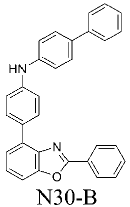 | 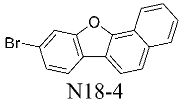 | 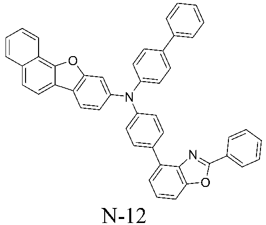 | 76% |
| 15 | 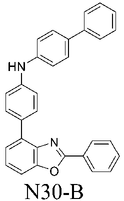 | 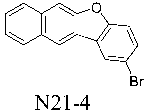 | 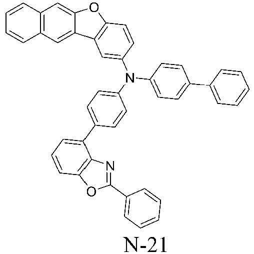 | 80% |
| 16 | 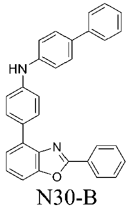 | 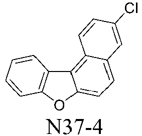 | 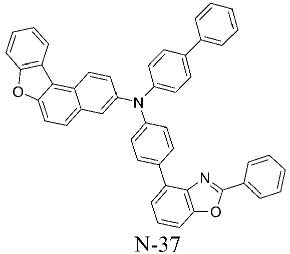 | 68% |
| 17 | 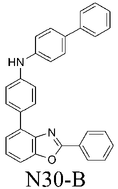 | 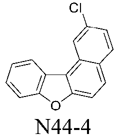 | 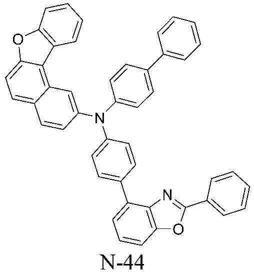 | 65% |
| 18 | 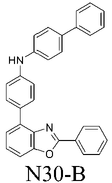 | 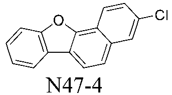 | 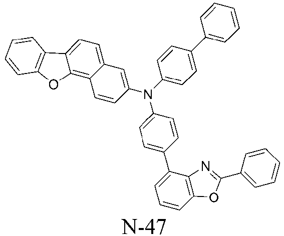 | 70% |
| 19 | 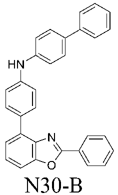 | 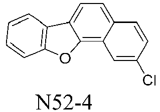 | 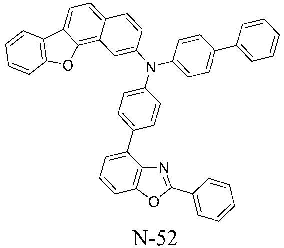 | 68% |
| 20 | 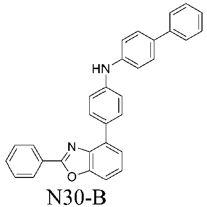 | 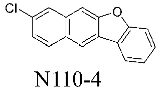 | 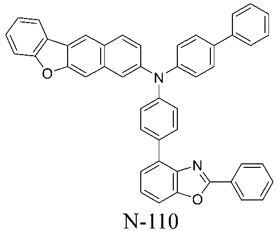 | 72% |
| 21 | 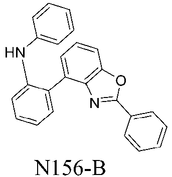 | 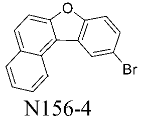 | 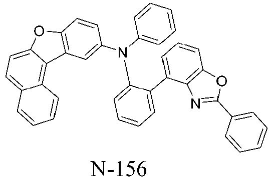 | |

The characterization data of the products prepared in Examples 13-21 are shown in Table 2:

**Table 2**

| Compound | Elemental analysis | | HRMS (ESI) m/z [M+H]+ | |
|---|---|---|---|---|
| | Theoretical value | Measured value | Theoretical value | Measured value |
| N-3 | C, 86.22; H, 4.62; N, 4.28; O, 4.89 | C, 86.24; H, 4.61; N, 4.27 | 654.23 | 655.16 |
| N-12 | C, 86.22; H, 4.62; N, 4.28; O, 4.89 | C, 86.23; H, 4.62; N, 4.27 | 654.23 | 655.32 |
| N-21 | C, 86.22; H, 4.62; N, 4.28; O, 4.89 | C, 86.24; H, 4.62; N, 4.26 | 654.23 | 655.35 |
| N-37 | C, 86.22; H, 4.62; N, 4.28; O, 4.89 | C, 86.20; H, 4.63; N, 4.29 | 654.23 | 654.78 |
| N-44 | C, 86.22; H, 4.62; N, 4.28; O, 4.89 | C, 86.25; H, 4.60; N, 4.27 | 654.23 | 654.98 |
| N-47 | C, 86.22; H, 4.62; N, 4.28; O, 4.89 | C, 86.24; H, 4.61; N, 4.27 | 654.23 | 654.93 |
| N-52 | C, 86.22; H, 4.62; N, 4.28; O, 4.89 | C, 86.20; H, 4.63; N, 4.29 | 654.23 | 654.82 |
| N-110 | C, 86.22; H, 4.62; N, 4.28; O, 4.89 | C, 86.21; H, 4.61; N, 4.28 | 654.23 | 654.97 |
| N-156 | C, 85.10; H, 4.53; N, 4.84; O, 5.53 | C, 85.12; H, 4.55; N, 4.80 | 578.20 | 579.26 |

### Example 22

This example provides an organic electroluminescent compound **M-3** in the luminescent host material, and the synthesis of organic electroluminescent compound **M-3** specifically includes the following steps:

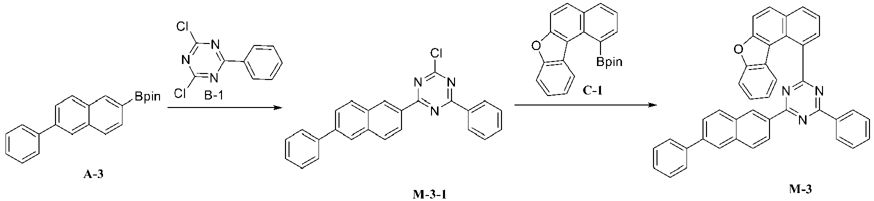

A 100 mL three-necked round-bottom flask equipped with a stirrer and a top-mounted reflux condenser was used. Under nitrogen protection, raw material A-3 (1 mmol), 2,4-dichloro-6-phenyl-1,3,5-triazine (1.2 mmol), Pd(dppf)Cl₂ (0.03 mmol), potassium carbonate (2.5 mmol), 1,4-dioxane/water (10 mL/2 mL) were added in sequence. The flask was heated to 60 ° C and the reaction was performed for 5 hours. After the reaction was completed, it was lowered to room temperature, and the reaction was quenched with saturated ammonium chloride aqueous solution, the material was extracted with ethyl acetate, the organic phase was dried with anhydrous magnesium sulfate, the solvent was removed using a rotary evaporator, and the crude product was separated using column chromatography (ethyl acetate: n-hexane = 1:50) to obtain intermediate M-3-1 (Yield 61%).

A 100 mL three-necked round-bottom flask equipped with a stirrer and a top-mounted reflux condenser was used. Under nitrogen protection, intermediate M-3-1 (1 mmol), 4,4,5,5-tetramethyl-2-(naphtho[2,1-b]benzofuran-1-yl)-1,3,2-dioxaborane (i.e., C-1, 1.2 mmol), Pd(dppf)Cl₂ (0.03 mmol), potassium carbonate (2.5 mmol), 1,4-dioxane/water (10 mL/2 mL) were added in sequence. The mixture was heated to 90 ° C and reacted for 5 hours. After the reaction was completed, it was cooled down to room temperature and filtered, the filter cake was rinsed twice with deionized water, and rinsed twice with ethanol to obtain a crude product, and the obtained crude product was purified twice with o-dichlorobenzene recrystallization, to obtain organic electroluminescent compound M-3 (yield 42%).

Elemental analysis: C₄₁H₂₅N₃. Theoretical value: C, 85.54; H, 4.38; N, 7.30; O, 2.78; measured value: C, 85.48; H, 4.50; N, 7.24. HRMS (ESI) m/z [M+H]⁺: theoretical value: 575.20; measured value: 576.20.

### Example 23

This example provides an organic electroluminescent compound **M-54** in the luminescent host material, and the synthesis of the organic electroluminescent compound **M-54** specifically includes the following steps:

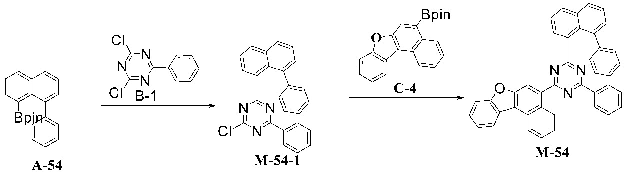

A 100 mL three-necked round-bottom flask equipped with a stirrer and a top-mounted reflux condenser was used. Under nitrogen protection, raw material A-54 (1 mmol), 2,4-dichloro-6-phenyl-1,3,5-triazine (1.2 mmol), Pd(dppf)Cl₂ (0.03 mmol), potassium carbonate (2.5 mmol), 1,4-dioxane/water (10 mL/2 mL) were added in sequence. The mixture was heated to 60°C and reacted for 5 hours. After the reaction was completed, the temperature was lowered to room temperature, the reaction was quenched with saturated ammonium chloride aqueous solution, the material was extracted with ethyl acetate, the organic phase was dried with anhydrous magnesium sulfate, the solvent was removed using a rotary evaporator to obtain a crude product, and the crude product was separated using column chromatography (ethyl acetate: n-hexane = 1:50) to obtain intermediate M-54-1 (yield 45%).

A 100 mL three-necked round-bottom flask equipped with a stirrer and a top-mounted reflux condenser was used. Under nitrogen protection, intermediate M-54-1 (1 mmol), 4,4,5,5-tetramethyl-2-(naphtho[2,1-b]benzofuran-5-yl)-1,3,2-dioxaborane (i.e., C-4, 1.2 mmol), Pd(dppf)Cl₂ (0.03 mmol), potassium carbonate (2.5 mmol), 1,4-dioxane/water (10 mL/2 mL) were added in sequence. The mixture was heated to 90°C and reacted for 5 hours. After the reaction was completed, the temperature was lowered to room temperature, the material was filtered, rinsed to obtain a filter cake, which was rinsed twice with deionized water, rinsed twice with ethanol to obtain a crude product, which was purified twice with o-dichlorobenzene recrystallization to obtain organic electroluminescent compound 54 (yield 46%).

Elemental analysis: C₄₁H₂₅N₃. Theoretical value: C, 85.54; H, 4.38; N, 7.30; O, 2.78; measured value: C, 85.44; H, 4.54; N, 7.24. HRMS (ESI) m/z [M+H]⁺: theoretical value: 575.20; measured value: 576.20.

### Example 24

This example provides an organic electroluminescent compound **M-58** in the luminescent host material, and the synthesis of the organic electroluminescent compound **M-58** specifically includes the following steps:

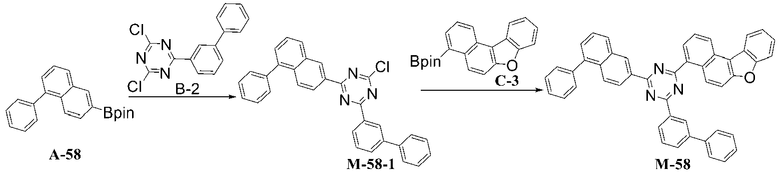

A 100 mL three-necked round-bottom flask equipped with a stirrer and a top-mounted reflux condenser was used. Under nitrogen protection, raw materials A-58 (1 mmol), 2-([1,1-biphenyl]-3-yl)-4,6-dichloro-1,3,5-triazine (i.e., B-2, 1.2 mmol), Pd(dppf)Cl₂ (0.03 mmol), potassium carbonate (2.5 mmol), 1,4-dioxane/water (10 mL/2 mL) were added in sequence. The mixture was heated to 60°C and reacted for 5 hours. After the reaction was completed, the temperature was lowered to room temperature, the reaction was quenched with saturated ammonium chloride aqueous solution, the material was extracted with ethyl acetate, the organic phase was dried with anhydrous magnesium sulfate, the solvent was removed using a rotary evaporator, and the crude product was separated using column chromatography (ethyl acetate: n-hexane = 1:50) to obtain intermediate M-59-1 (yield 54%).

A 100 mL three-necked round-bottom flask equipped with a stirrer and a top-mounted reflux condenser was used. Under nitrogen protection, intermediate M-59-1 (1 mmol), 4,4,5,5-tetramethyl-2-(naphtho[2,1-b]benzofuran-2-yl)-1,3,2-dioxaborane (i.e., C-3, 1.2 mmol), Pd(dppf)Cl₂ (0.03 mmol), potassium carbonate (2.5 mmol), 1,4-dioxane/water (10 mL/2 mL) were added in sequence. The mixture was heated to 90°C and reacted for 5 hours. After the reaction was completed, the temperature was lowered to room temperature, the material was filtered, the filter cake was rinsed twice with deionized water, rinsed twice with ethanol, and the crude product was purified twice with o-dichlorobenzene recrystallization to obtain organic electroluminescent compound M-58 (yield 71%).

Elemental analysis: C₄₇H₂₉N₃. Theoretical value: C, 86.61; H, 4.49; N, 6.45; O, 2.45; measured value: C, 86.53; H, 4.59; N, 6.41. HRMS (ESI) m/z [M+H]⁺: theoretical value: 651.23; measured value: 652.24.

### Example 25

This example provides an organic electroluminescent compound **M-66** in the luminescent host material, and the synthesis of the organic electroluminescent compound **M-66** specifically includes the following steps:

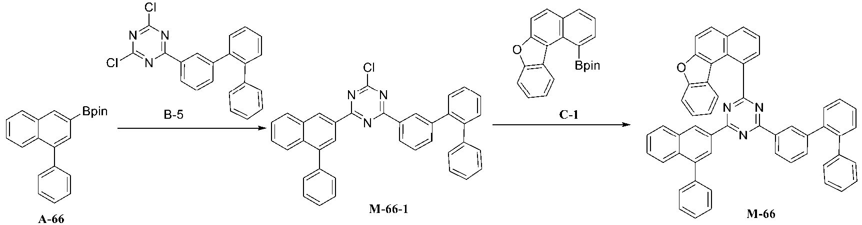

A 100 mL three-necked round-bottom flask equipped with a stirrer and a top-mounted reflux condenser was used. Under nitrogen protection, raw materials A-66 (1 mmol), 2-([1,1:2,1-triphenyl]-3-yl)-4,6-dichloro-1,3,5-triazine (i.e., B-5, 1.2 mmol), Pd(dppf)Cl₂ (0.03 mmol), potassium carbonate (2.5 mmol), 1,4-dioxane/water (10 mL/2 mL) were added in sequence. The mixture was heated to 60°C and reacted for 5 hours. After the reaction was completed, the temperature was lowered to room temperature, the reaction was quenched with saturated ammonium chloride aqueous solution, the material was extracted with ethyl acetate, the organic phase was dried with anhydrous magnesium sulfate, the solvent was removed using a rotary evaporator, and the crude product was separated by column chromatography (ethyl acetate: n-hexane = 1:50) to obtain intermediate M-66-1 (yield 49%).

A 100 mL three-necked round-bottom flask equipped with a stirrer and a top-mounted reflux condenser was used. Under nitrogen protection, intermediate M-66-1 (1 mmol), 4,4,5,5-tetramethyl-2-(naphtho[2,1-b]benzofuran-2-yl)-1,3,2-dioxaborane (i.e., C-1, 1.2 mmol), Pd(dppf)Cl₂ (0.03 mmol), potassium carbonate (2.5 mmol), 1,4-dioxane/water (10 mL/2 mL) were added in sequence. The mixture was heated to 90°C and reacted for 5 hours. After the reaction was completed, the temperature was lowered to room temperature, the material was filtered, the filter cake was rinsed twice with deionized water, rinsed twice with ethanol, and the crude product was purified twice with o-dichlorobenzene recrystallization to obtain Compound M-66 (yield 57%).

Elemental analysis: C₅₃H₃₃N₃O. Theoretical value: C, 87.46; H, 4.57; N, 5.77; O, 2.20; measured value: C, 87.33; H, 4.79; N, 5.68. HRMS (ESI) m/z [M+H]⁺: theoretical value: 727.26; measured value: 728.24.

The preparation method of Examples 26-33 is similar to Example 22. Specifically, the raw materials used and the products prepared in Examples 26-33 are shown in Table 3.

**Table 3**

| Example | Raw material M-n-1 | Raw material C-n | Product | Yield% |
|---|---|---|---|---|
| 26 | 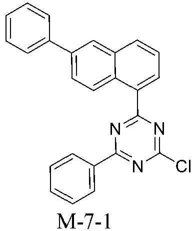 | 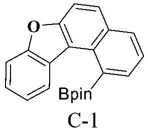 | 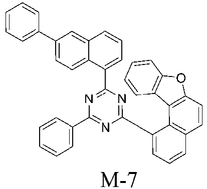 | 63 |
| 27 | 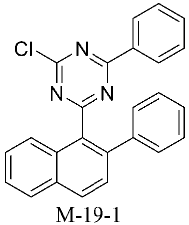 | 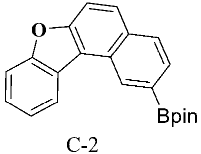 | 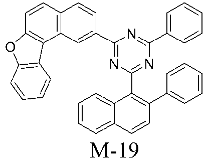 | 58 |
| 28 | 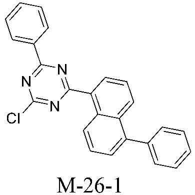 | 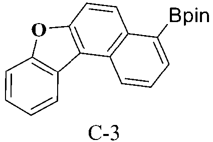 | 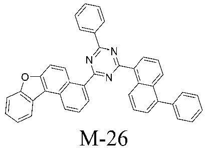 | 49 |
| 29 | 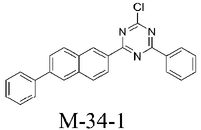 | 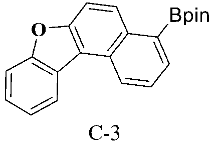 | 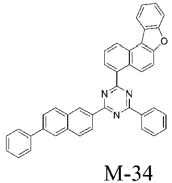 | 71 |
| 30 | 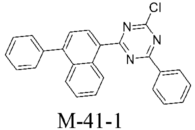 | 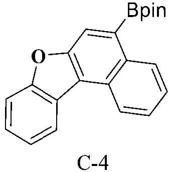 | 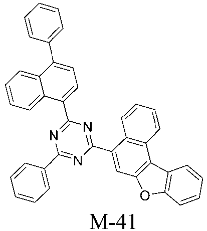 | 65 |
| 31 | 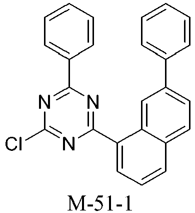 | 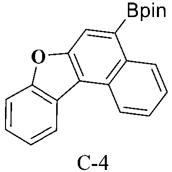 | 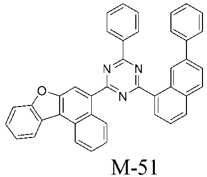 | 61 |
| 32 | 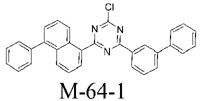 | 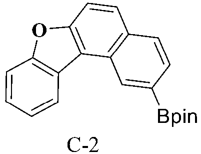 | 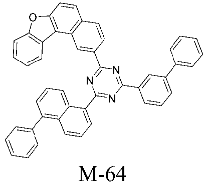 | 57 |
| 33 | 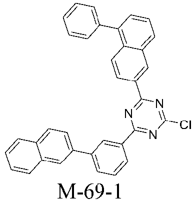 | 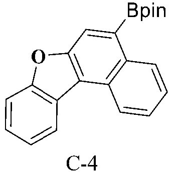 | 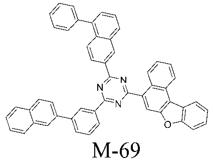 | 46 |

The characterization data of the products prepared in Examples 26-33 are shown in Table 4.

**Table 4**

| Compound | Elemental analysis | | HRMS (ESI) m/z [M+H]+ | |
|---|---|---|---|---|
| | Theoretical value | Measured value | Theoretical value | Measured value |
| M-7 | C, 85.54; H, 4.38; N, 7.30; O, 2.78 | C, 85.64; H, 4.48; N, 7.10 | 575.20 | 576.20 |
| M-19 | C, 85.54; H, 4.38; N, 7.30; O, 2.78 | C, 85.63; H, 4.49; N, 7.10 | 575.20 | 576.20 |
| M-26 | C, 85.54; H, 4.38; N, 7.30; O, 2.78 | C, 85.65; H, 4.47; N, 7.10 | 575.20 | 576.20 |
| M-34 | C, 85.54; H, 4.38; N, 7.30; O, 2.78 | C, 85.62; H, 4.49; N, 7.11 | 575.20 | 576.20 |
| M-41 | C, 85.54; H, 4.38; N, 7.30; O, 2.78 | C, 85.61; H, 4.49; N, 7.12 | 575.20 | 576.20 |
| M-41 | C, 85.54; H, 4.38; N, 7.30; O, 2.78 | C, 85.62; H, 4.47; N, 7.13 | 575.20 | 576.20 |
| M-64 | C, 85.54; H, 4.38; N, 7.30; O, 2.78 | C, 85.64; H, 4.47; N, 7.11 | 575.20 | 576.20 |
| M-69 | C, 87.28; H, 4.45; N, 5.99; O, 2.28 | C, 87.38; H, 4.55; N, 5.79 | 701.25 | 702.15 |

### Device Example

The present example provides an organic electroluminescent device, as shown in FIG. 1, comprising an anode 2, a hole injection layer 3, a hole transport layer 4, a light-emitting layer 5, an electron transport layer 6, an electron injection layer 7 and a cathode 8 stacked in sequence on a substrate 1, and the device structure is: anode (indium tin oxide (ITO) coated glass substrate)/hole injection layer (HIL)/hole transport layer (HTL)/light-emitting layer (EML)/electron transport layer (ETL)/electron injection layer (EIL)/cathode (Al).

The materials for manufacturing the organic electroluminescent device are as follows:

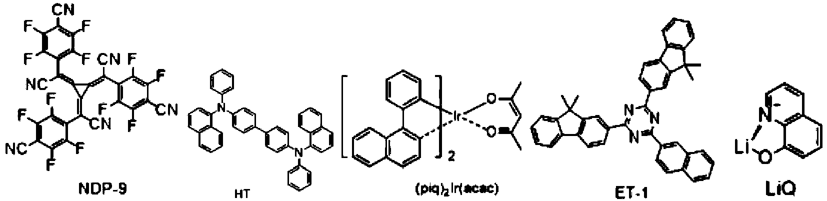

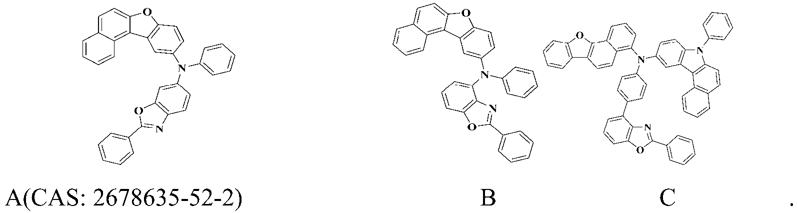

The preparation method of compound B is the same as that of the benzonaphthofuran-containing oxazole organic compound N-1, except that the 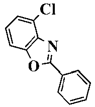 is used to replace intermediate N1-A to obtain compound B with a yield of 64%.

Elemental analysis: C₃₅H₂₂N₂O₂; theoretical value: C, 83.65; H, 4.41; N, 5.57; O, 6.37; measured value: C, 83.62; H, 4.41; N, 5.61. HRMS (ESI) m/z [M+H]+: theoretical value: 502.17; measured value: 503.32.

The preparation method of compound C is the same as that of the organic electroluminescent compound N-1, except that 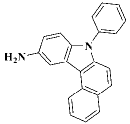 is used to replace raw material N1-3, and 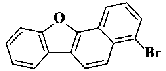 is used to replace raw material N1-4 to obtain compound C with a yield of 60%.

Elemental analysis: C₅₇H₃₅N₃O₂; theoretical value: C, 86.23; H, 4.44; N, 5.29; O, 4.03; measured value: C, 86.28; H, 4.47; N, 5.21. HRMS (ESI) m/z [M+H]+: theoretical value: 793.27; measured value: 794.25.

The preparation of the organic electroluminescent device comprises the following steps:

### 1) Substrate cleaning:

The glass substrate coated with transparent ITO was ultrasonically treated in an aqueous cleaning agent (the composition and concentration of the aqueous cleaning agent: ethylene glycol solvent ≤ 10wt%, triethanolamine ≤ 1wt%), then rinsed in deionized water, ultrasonically degreased in a mixed solvent of acetone and ethanol (the volume ratio of acetone to ethanol is 1:1), baked in a clean environment until the moisture was completely removed, and then cleaned with ultraviolet light and ozone.

### 2) Preparation of organic layer:

The ITO transparent substrate was transferred to the evaporation equipment and evacuated to 1×10⁻⁶ to 2×10⁻⁴ Pa, and the hole injection layer (HIL)/hole transport layer (HTL)/ light-emitting layer (EML)/electron transport layer (ETL)/electron injection layer (EIL)/thick cathode (Al) were sequentially evaporated on the anode film;
wherein the material of the hole injection layer (HIL) is a mixture of NDP-9 and HT, and the specific mass ratio is shown in Table 5;
The materials of the hole transport layer (HTL) are shown in Table 5;
The light-emitting layer (EML) is vacuum evaporated by co-evaporation. The material of the light-emitting layer includes a host material and a guest material, wherein the guest material is (piq)₂Ir(acac). The specific material of the host material and the ratio of the host material to the guest material are shown in Table 5.
The materials of the electron transport layer (ETL) are shown in Table 5;
The material of the electron injection layer (EIL) is LiQ;
The cathode is aluminum;

Some layers of the organic electroluminescent device and their materials and thicknesses are shown in Table 5:

**Table 5**

| Example No. | HIL thickness | HTL thickness | EML thickness | ETL thickness | EIL thickness | Cathode thickness |
|---|---|---|---|---|---|---|
| Example 1 | NDP-9: HT (mass ratio 3:97) 10 nm | HT 80 nm | M-3: N-1: (piq)21r(acac) (mass ratio 47.5:47.5:5) 38 nm | ET-1: LiQ (mass ratio 1:1) 30 nm | LiQ 1 nm | Al 80 nm |
| Example 2 | NDP-9: HT (mass ratio 3:97) 10 nm | HT 80 nm | M-3: N-3: (piq)2Ir(acac) (mass ratio 47.5:47.5:5) 38 nm | ET-1: LiQ (mass ratio 1:1) 30 nm | LiQ 1 nm | Al 80 nm |
| Example 3 | NDP-9: HT (mass ratio 3:97) 10 nm | HT 80 nm | M-7: N-6: (piq)2Ir(acac) (mass ratio 47.5:47.5:5) 38 nm | ET-1: LiQ (mass ratio 1:1) 30 nm | LiQ 1 nm | Al 80 nm |
| Example 4 | NDP-9: HT (mass ratio 3:97) 10 nm | HT 80 nm | M-19:N-12:(piq)2Ir(acac) (mass ratio 47.5:47.5:5) 38 nm | ET-1: LiQ (mass ratio 1:1) 30 nm | LiQ 1 nm | Al 80 nm |
| Example 5 | NDP-9: HT (mass ratio 3:97) 10 nm | HT 80 nm | M-26:N-18:(piq)21r(acac) (mass ratio 47.5:47.5:5) 38 nm | ET-1: LiQ (mass ratio 1: 1) 30 nm | LiQ 1 nm | Al 80 nm |
| Example 6 | NDP-9: HT (mass ratio 3:97) 10 nm | HT 80 nm | M-7:N-21:(piq)2Ir(acac) (mass ratio 47.5:47.5:5) 38 nm | ET-1: LiQ (mass ratio 1:1) 30 nm | LiQ 1 nm | Al 80 nm |
| Example 7 | NDP-9: HT (mass ratio 3:97) 10 nm | HT 80 nm | M-26:N-30:(piq)2Ir(acac) (mass ratio 47.5:47.5:5) 38 nm | ET-1: LiQ (mass ratio 1:1) 30 nm | LiQ 1 nm | Al 80 nm |
| Example 8 | NDP-9: HT (mass ratio 3:97) 10 nm | HT 80 nm | M-34:N-37:(piq)2Ir(acac) (mass ratio 47.5:47.5:5) 38 nm | ET-1: LiQ (mass ratio 1:1) 30 nm | LiQ 1 nm | Al 80 nm |
| Example 9 | NDP-9: HT (mass ratio 3:97) 10 nm | HT 80 nm | M-41:N-44:(piq)2Ir(acac) (mass ratio 47.5:47.5:5) 38 nm | ET-1: LiQ (mass ratio 1:1) 30 nm | LiQ 1 nm | Al 80 nm |
| Example 10 | NDP-9: HT (mass ratio 3:97) 10 nm | HT 80 nm | M-64:N-45:(piq)21r(acac) (mass ratio 47.5:47.5:5) 38 nm | ET-1: LiQ (mass ratio 1:1) 30 nm | LiQ 1 nm | Al 80 nm |
| Example 11 | NDP-9: HT (mass ratio 3:97) 10 nm | HT 80 nm | M-64:N-47:(piq)2Ir(acac) (mass ratio 47.5:47.5:5) 38 nm | ET-1: LiQ (mass ratio 1:1) 30 nm | LiQ 1 nm | Al 80 nm |
| Example 12 | NDP-9: HT (mass ratio 3:97) 10 nm | HT 80 nm | M-64:N-72:(piq)2Ir(acac) (mass ratio 47.5:47.5:5) 38 nm | ET-1: LiQ (mass ratio 1:1) 30 nm | LiQ 1 nm | Al 80 nm |
| Example 13 | NDP-9: HT (mass ratio 3:97) 10 nm | HT 80 nm | M-64:N-95:(piq)2Ir(acac) (mass ratio 47.5:47.5:5) 38 nm | ET-1: LiQ (mass ratio 1:1) 30 nm | LiQ 1 nm | Al 80 nm |
| Example 14 | NDP-9: HT (mass ratio 3:97) 10 nm | HT 80 nm | M-64:N-110:(piq)2Ir(acac)(mass ratio 47.5:47.5:5) 38 nm | ET-1: LiQ (mass ratio 1:1) 30 nm | LiQ 1 nm | Al 80 nm |
| Example 15 | NDP-9: HT (mass ratio 3:97) 10 nm | HT 80 nm | M-3:N-134:(piq)2Ir(acac) (mass ratio 47.5:47.5:5) 38 nm | ET-1: LiQ (mass ratio 1:1) 30 nm | LiQ 1 nm | Al 80 nm |
| Example 16 | NDP-9: HT (mass ratio 3:97) 10 nm | HT 80 nm | M-3:N-148:(piq)2Ir(acac) (mass ratio 47.5:47.5:5) 38 nm | ET-1: LiQ (mass ratio 1:1) 30 nm | LiQ 1 nm | Al 80 nm |
| Example 17 | NDP-9: HT (mass ratio 3:97) 10 nm | HT 80 nm | M-3:N-156:(piq)2Ir(acac) (mass ratio 47.5:47.5:5) 38 nm | ET-1: LiQ (mass ratio 1:1) 30 nm | LiQ 1 nm | Al 80 nm |
| Example 18 | NDP-9: HT (mass ratio 3:97) 10 nm | HT 80 nm | M-3:N-173:(piq)2Ir(acac) (mass ratio 47.5:47.5:5) 38 nm | ET-1: LiQ (mass ratio 1:1) 30 nm | LiQ 1 nm | Al 80 nm |
| Example 19 | NDP-9: HT (mass ratio 3:97) 10 nm | HT 80 nm | M-3:N-182:(piq)2Ir(acac) (mass ratio 47.5:47.5:5) 38 nm | ET-1: LiQ (mass ratio 1:1) 30 nm | LiQ 1 nm | Al 80 nm |
| Example 20 | NDP-9: HT (mass ratio 3:97) 10 nm | HT 80 nm | M-3:N-207:(piq)21r(acac) (mass ratio 47.5:47.5:5) 38 nm | ET-1: LiQ (mass ratio 1:1) 30 nm | LiQ 1 nm | Al 80 nm |
| Example 21 | NDP-9: HT (mass ratio 3:97) 10 nm | HT 80 nm | M-3:(piq)2Ir(acac) (mass ratio 95:5) 38 nm | ET-1: LiQ (mass ratio 1:1) 30 nm | LiQ 1 nm | Al 80 nm |
| Example 22 | NDP-9: HT (mass ratio 3:97) 10 nm | HT 80 nm | M-64:(piq)2Ir(acac) (mass ratio 95:5) 38 nm | ET-1: LiQ (mass ratio 1: 1) 30 nm | LiQ 1 nm | Al 80 nm |
| Example 23 | NDP-9: HT (mass ratio 3:97) 10 nm | HT 80 nm | N3:(piq)2Ir(acac) (mass ratio 95:5) 38 nm | ET-1: LiQ (mass ratio 1: 1) 30 nm | LiQ 1 nm | Al 80 nm |
| Example 24 | NDP-9: HT (mass ratio 3:97) 10 nm | HT 80 nm | N-21:(piq)2Ir(acac) (mass ratio 95:5) 38 nm | ET-1: LiQ (mass ratio 1: 1) 30 nm | LiQ 1 nm | Al 80 nm |
| Comparative Example 1 | NDP-9: HT (mass ratio 3:97) 10 nm | HT 80 nm | A:(piq)2Ir(acac) (mass ratio 95:5) 38 nm | ET-1: LiQ (mass ratio 1: 1) 30 nm | LiQ 1 nm | Al 80 nm |
| Comparative Example 2 | NDP-9: HT (mass ratio 3:97) 10 nm | HT 80 nm | B:(piq)2Ir(acac) (mass ratio 95:5) 38 nm | ET-1: LiQ (mass ratio 1: 1) 30 nm | LiQ 1 nm | Al 80 nm |
| Comparative Example 3 | NDP-9: HT (mass ratio 3:97) 10 nm | HT 80 nm | C:(piq)2Ir(acac) (mass ratio 95:5) 38 nm | ET-1: LiQ (mass ratio 1: 1) 30 nm | LiQ 1 nm | Al 80 nm |
| Comparative Example 4 | NDP-9: HT (mass ratio 3:97) 10 nm | HT 80 nm | M-3:A:(piq)2Ir(acac) (mass ratio 47.5:47.5:5) 38 nm | ET-1: LiQ (mass ratio 1: 1) 30 nm | LiQ 1 nm | Al 80 nm |
| Comparative Example 5 | NDP-9: HT (mass ratio 3:97) 10 nm | HT 80 nm | M-3:B:(piq)2Ir(acac) (mass ratio 47.5:47.5:5) 38 nm | ET-1: LiQ (mass ratio 1: 1) 30 nm | LiQ 1 nm | Al 80 nm |
| Comparative Example 6 | NDP-9: HT (mass ratio 3:97) 10 nm | HT 80 nm | M-3:C:(piq)2Ir(acac) (mass ratio 47.5:47.5:5) 38 nm | ET-1: LiQ (mass ratio 1:1) 30 nm | LiQ 1 nm | Al 80 nm |

The examples in the table represent device examples, and the comparative examples in the table are device comparative examples.

### Test Example

The organic electroluminescent devices obtained from device examples 1 to 24 and comparative examples 1 to 6 in the device examples were tested.
Instruments: The current, voltage, brightness and other characteristics of the device were tested synchronously using a PR 650 spectrum scanning brightness meter and a Keithley K2400 digital source meter system;
Test conditions: Photoelectric characteristics test conditions: Current density was 10mA/cm².
Lifetime test: The current density was 50 mA/cm², and the time (in hours) when the device brightness drops to 95% of the original brightness was recorded.

The device performance test results are shown in Table 6:

**Table 6**

| No. | Driving voltage (V) | Current efficiency (Cd/A) | Lifespan T95 (hours) |
|---|---|---|---|
| Example 1 | 3.25 | 33.71 | 342.5 |
| Example 2 | 3.18 | 36.47 | 364.7 |
| Example 3 | 3.25 | 33.51 | 335.4 |
| Example 4 | 3.21 | 33.54 | 342.5 |
| Example 5 | 3.30 | 32.45 | 318.7 |
| Example 6 | 3.23 | 33.41 | 326.8 |
| Example 7 | 3.32 | 32.18 | 315.4 |
| Example 8 | 3.18 | 36.54 | 354.8 |
| Example 9 | 3.18 | 35.27 | 360.7 |
| Example 10 | 3.23 | 32.27 | 322.4 |
| Example 11 | 3.20 | 34.52 | 320.5 |
| Example 12 | 3.23 | 32.86 | 325.4 |
| Example 13 | 3.35 | 33.25 | 326.8 |
| Example 14 | 3.25 | 33.96 | 330.3 |
| Example 15 | 3.36 | 31.02 | 315.7 |
| Example 16 | 3.23 | 32.11 | 320.8 |
| Example 17 | 3.23 | 33.66 | 340.7 |
| Example 18 | 3.28 | 32.18 | 325.1 |
| Example 19 | 3.24 | 32.58 | 327.5 |
| Example 20 | 3.26 | 31.49 | 310.8 |
| Example 21 | 3.66 | 25.47 | 174.2 |
| Example 22 | 3.62 | 25.64 | 188.5 |
| Example 23 | 3.89 | 15.62 | 65.4 |
| Example 24 | 3.92 | 12.11 | 41.5 |
| Comparative Example 1 | 4.05 | 8.65 | 22.7 |
| Comparative Example 2 | 4.01 | 10.35 | 35.4 |
| Comparative Example 3 | 4.21 | 7.10 | 20.4 |
| Comparative Example 4 | 3.71 | 28.15 | 286.7 |
| Comparative Example 5 | 3.52 | 30.24 | 312.8 |
| Comparative Example 6 | 3.87 | 22.51 | 212.7 |

The examples in the table represent device examples, and the comparative examples in the table are device comparative examples.

Obviously, the above examples are merely examples for clear description and are not limitations of the implementation methods. For those skilled in the art, other different forms of changes or modifications can be made based on the above description. It is not necessary and impossible to list all implementation methods exhaustively here. The obvious changes or modifications derived therefrom are still within the protection scope of the present invention.

## Claims

1. A benzonaphthofuran-containing oxazole organic compound, wherein the oxazole organic compound has a structure represented by Formula (1): 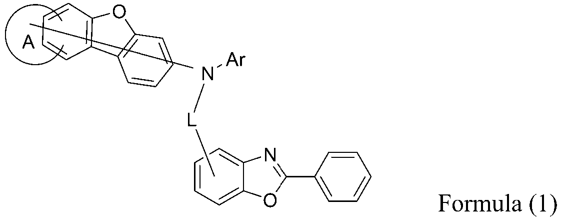
in Formula (1), ring A is a benzene ring;
Ar is selected from the group consisting of substituted or unsubstituted C6-C60 aryl, substituted or unsubstituted C6-C60 arylamine, substituted or unsubstituted C3-C60 heteroarylamine, and substituted or unsubstituted C3-C30 heteroaryl;
L is selected from the group consisting of substituted or unsubstituted C6-C30 arylene, and substituted or unsubstituted C3-C30 heteroarylene;
the substituents in the substituted C6-C60 aryl, substituted C6-C60 arylamine, substituted C3-C60 heteroarylamine, substituted C3-C30 heteroaryl, substituted C6-C30 arylene, and substituted C3-C30 heteroarylene are each independently selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof.

2. The benzonaphthofuran-containing oxazole organic compound according to claim 1, wherein Ar is selected from the group consisting of substituted or unsubstituted C6-C25 aryl, substituted or unsubstituted C6-C25 arylamine, substituted or unsubstituted C3-C25 heteroarylamine, and substituted or unsubstituted C3-C20 heteroaryl;
wherein the substituents in the substituted C6-C25 aryl, substituted C6-C25 arylamine, substituted C3-C25 heteroarylamine, and substituted C3-C20 heteroaryl are each independently selected from the group consisting of deuterium, halogen, cyano, C1-C12 alkyl, C3-C12 cycloalkyl, C6-C30 aryl, C3-30 heteroaryl, C6-C60 arylamine and C3-C60 heteroarylamine, or a combination of at least two thereof;
optionally, Ar is selected from substituted or unsubstituted B group, and the B group is selected from the group consisting of phenyl, naphthyl, biphenyl, phenanthryl, fluoranthenyl, chrysenyl, terphenyl, triphenylenyl, phenalenyl, phenylnaphthyl, naphthylphenyl, dimethylfluorenyl, phenylmethylfluorenyl, diphenylfluorenyl, pyridyl, pyridylphenyl, phenylpyridyl, spirobifluorenyl, benzodimethylfluorenyl, benzodiphenylfluorenyl, benzospirobifluorenyl, dibenzofuryl, benzonaphthofuryl, benzonaphthothienyl, spiro[fluorene-9,9'-xanthene]-yl, phenylmethylfluorenyl, dinaphthofuranyl, dinaphthothiophenyl, dibenzothiophenyl, and N, N-diphenylanilino;
wherein the substituents of the substituted B group are selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof.

3. The benzonaphthofuran-containing oxazole organic compound according to claim 1 or 2, wherein Ar is selected from the group consisting of phenyl, naphthyl, biphenyl, chrysenyl, phenanthryl, terphenyl, phenylnaphthyl, naphthylphenyl, dimethylfluorenyl, diphenylfluorenyl, spirobifluorenyl, phenalenyl, dibenzofuryl, benzonaphthofuryl, and N, N-diphenylanilino.

4. The benzonaphthofuran-containing oxazole organic compound according to any one of claims 1 to 3, wherien L is selected from the group consisting of substituted or unsubstituted C6-C15 arylene; wherein the substituents in the substituted C6-C15 arylene are each independently selected from the group consisting of deuterium, halogen and C1-C62 alkyl, or a combination of at least two thereof;
optionally, L is selected from the group consisting of phenylene, biphenylene and naphthylene; and
optionally, L is selected from the group consisting of phenylene and naphthylene.

5. The benzonaphthofuran-containing oxazole organic compound according to any one of claims 1 to 4, wherein Formula (1) is selected from one of the structures represented by Formulas 1-1 to 1-6 below: 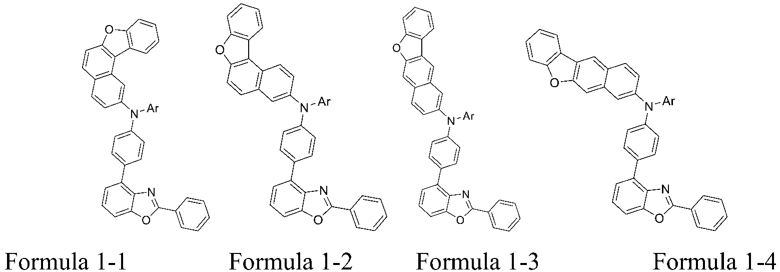 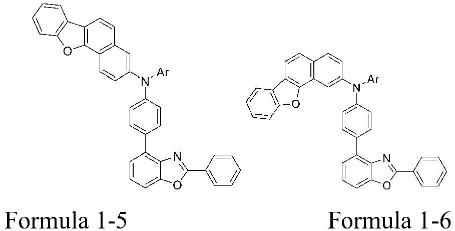 wherein the definition of Ar is the same as that in claim 1.

6. The benzonaphthofuran-containing oxazole organic compound according to any one of claims 1 to 5, wherein the benzonaphthofuran-containing oxazole organic compound is selected from any one of N-1 to N-208:

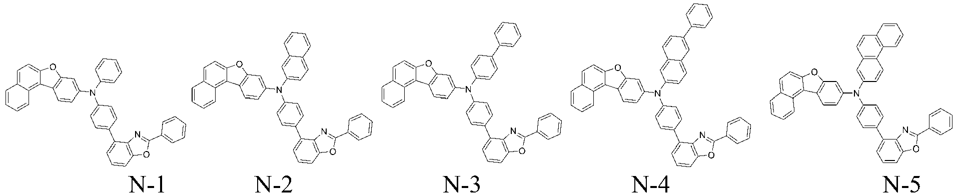

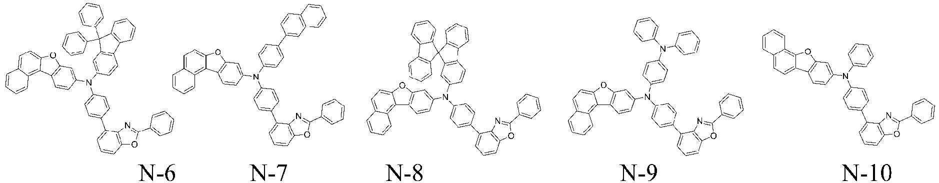

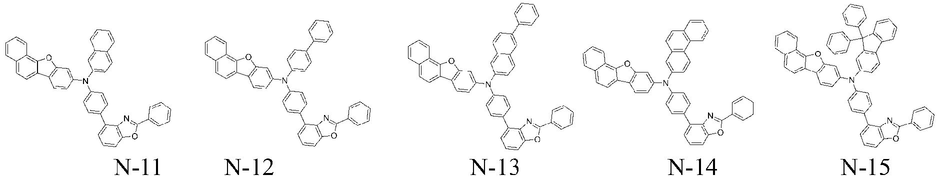

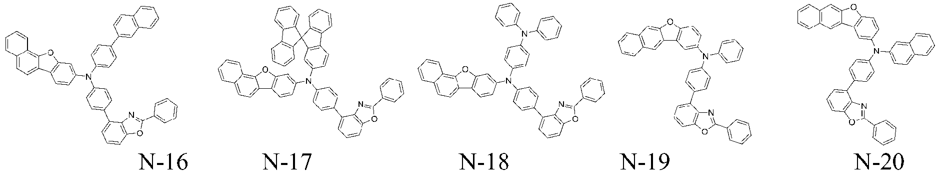

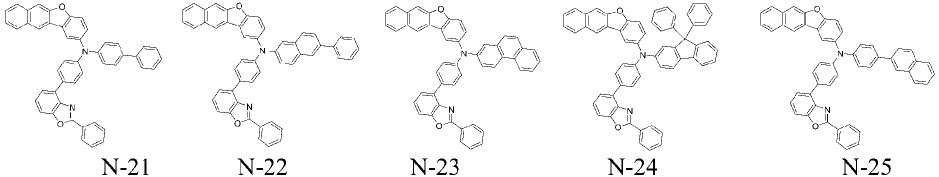

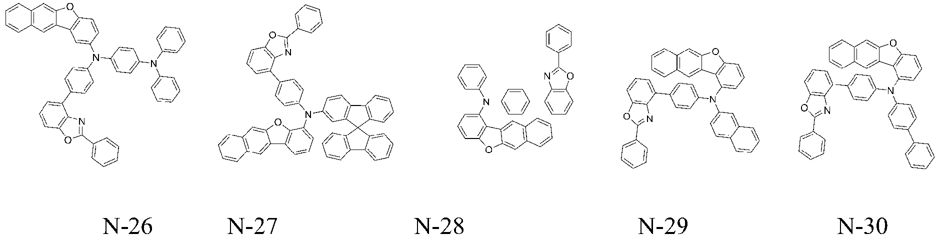

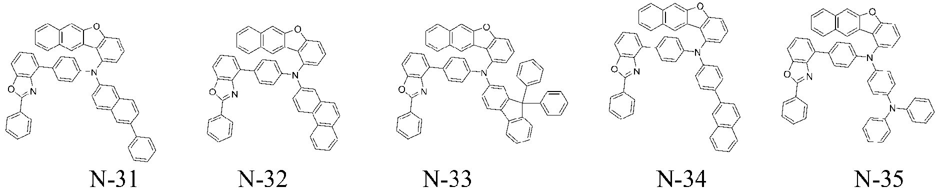

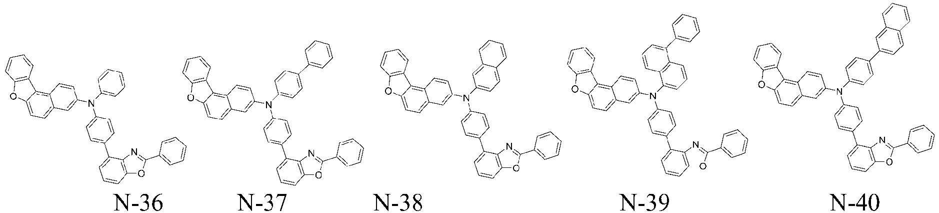

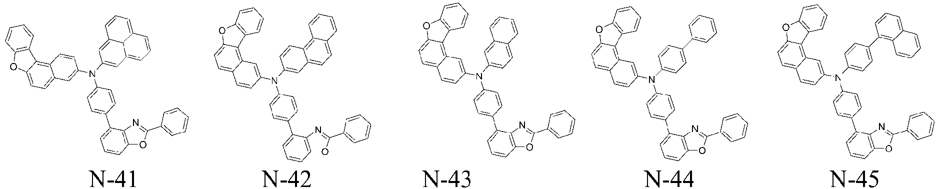

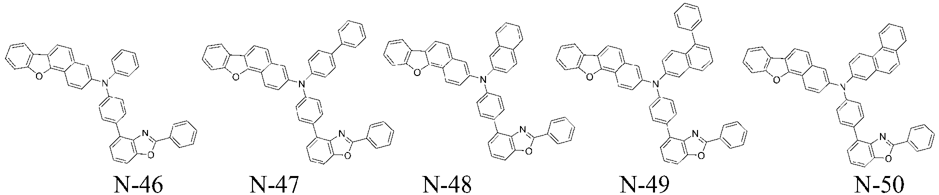

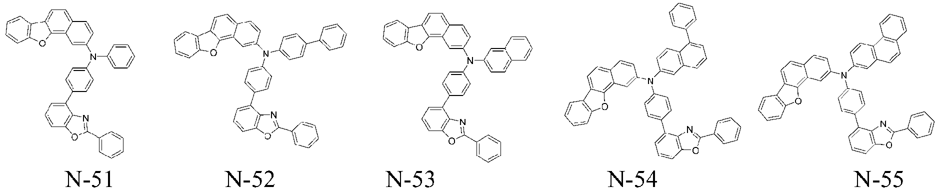

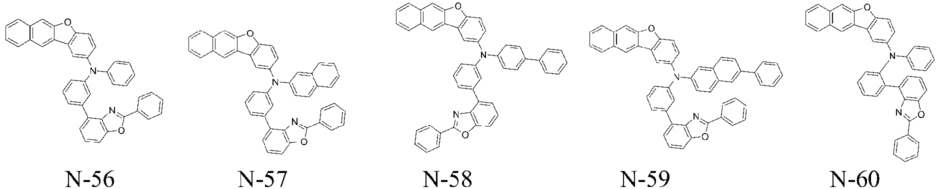

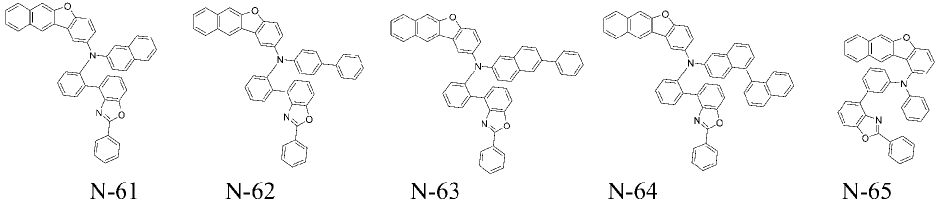

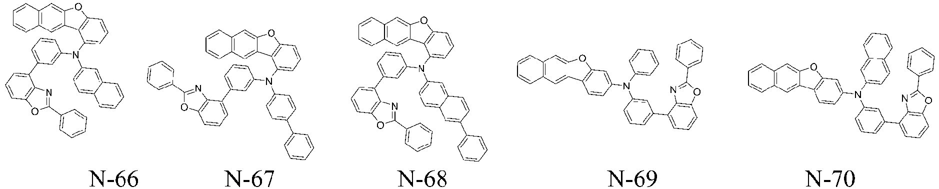

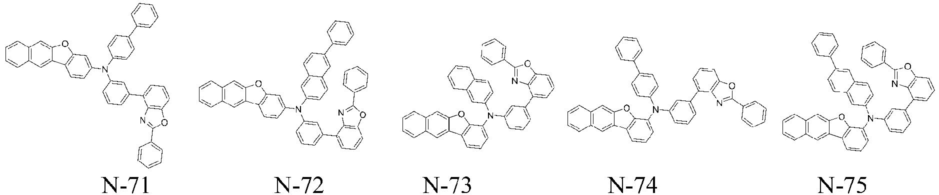

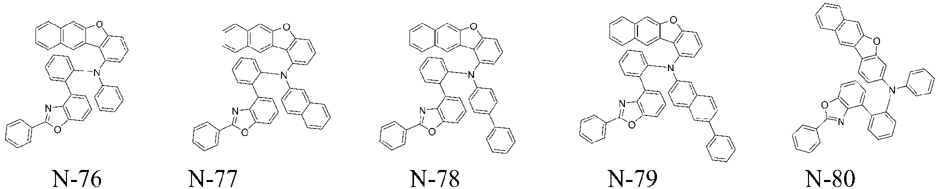

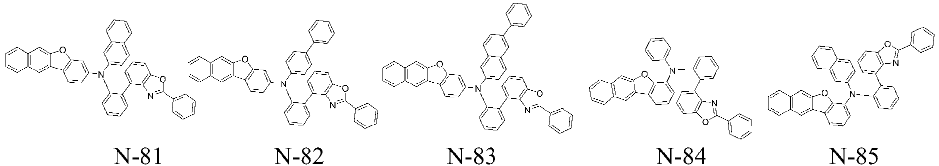

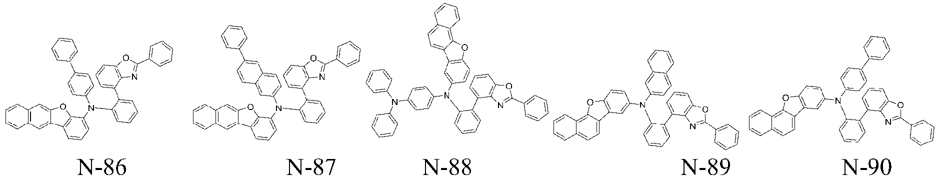

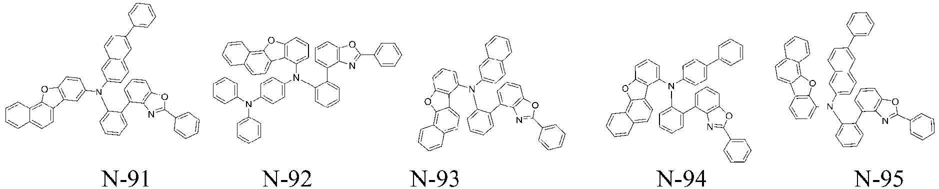

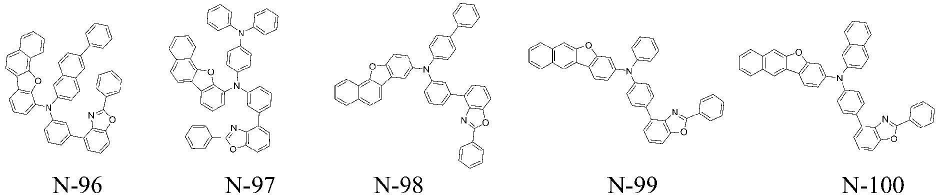

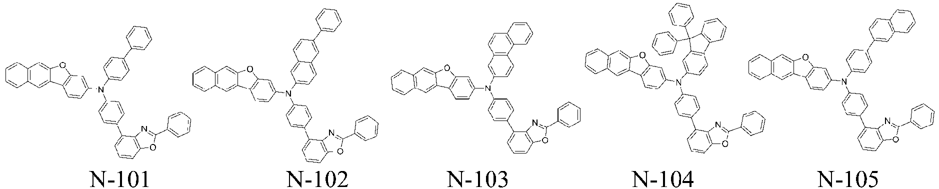

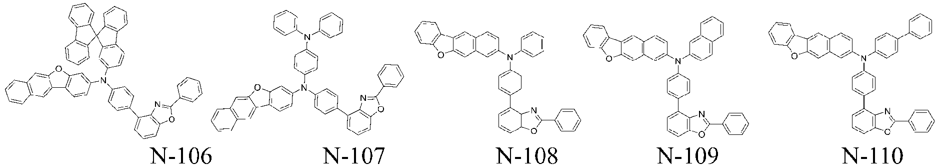

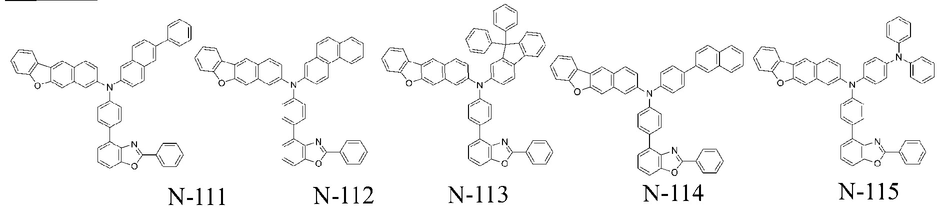

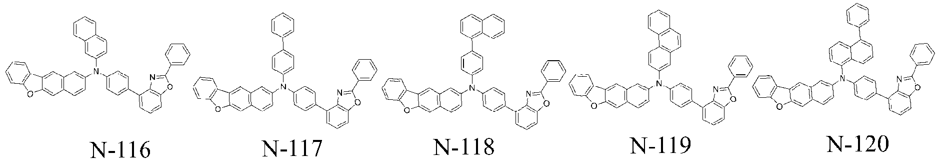

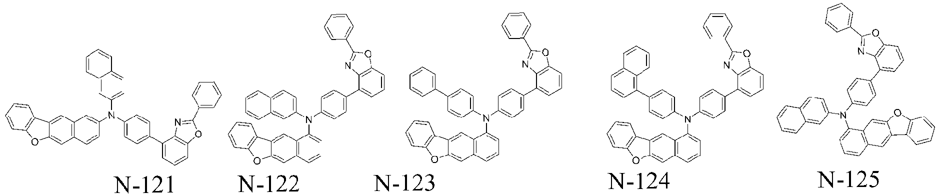

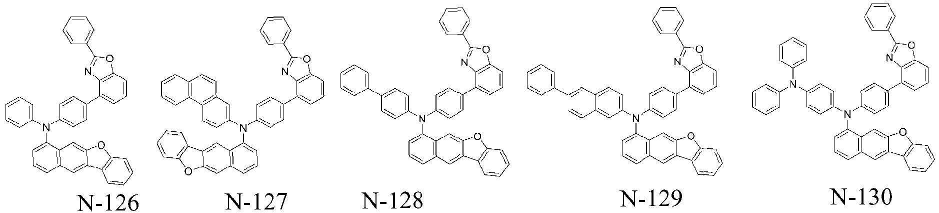

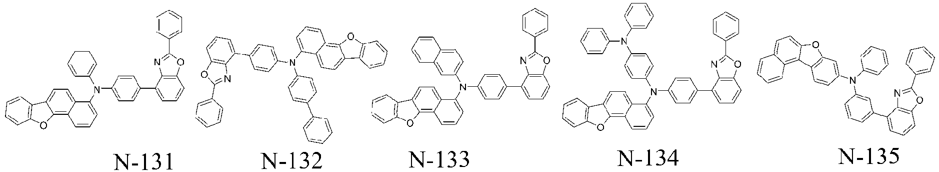

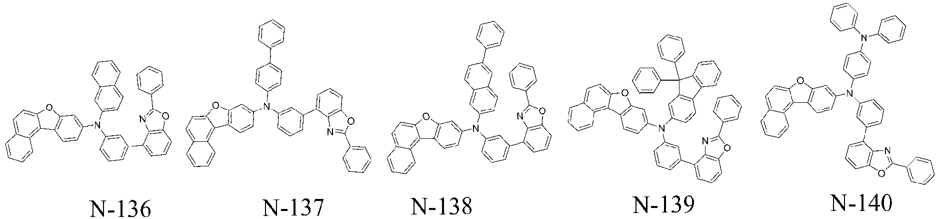

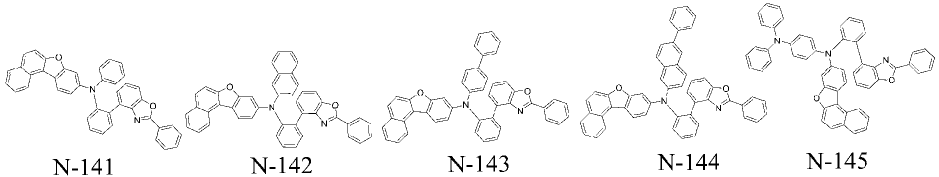

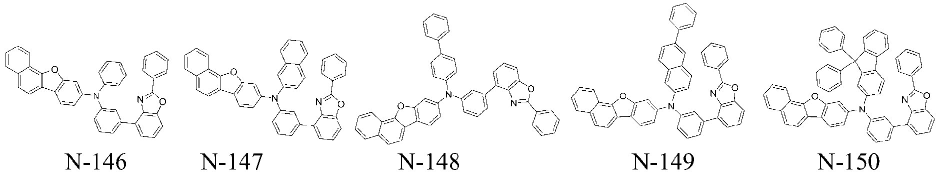

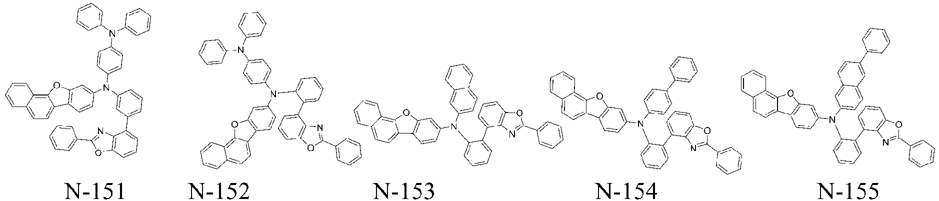

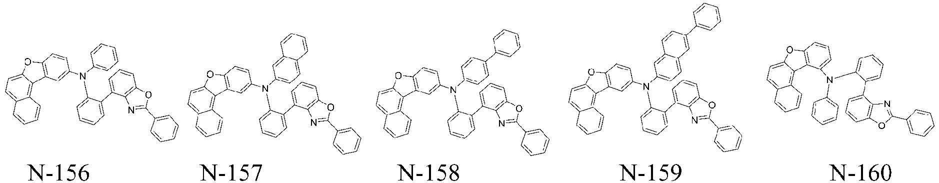

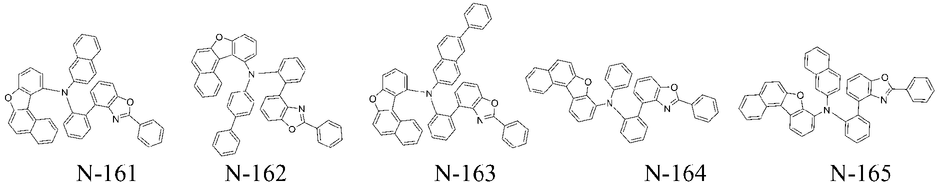

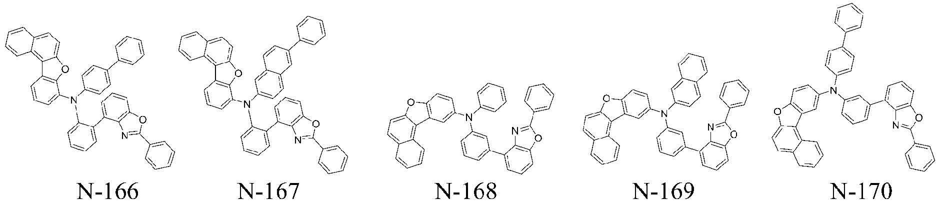

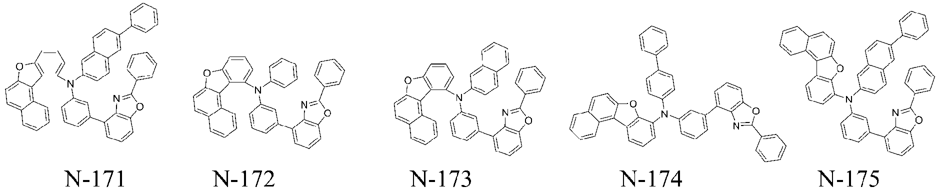

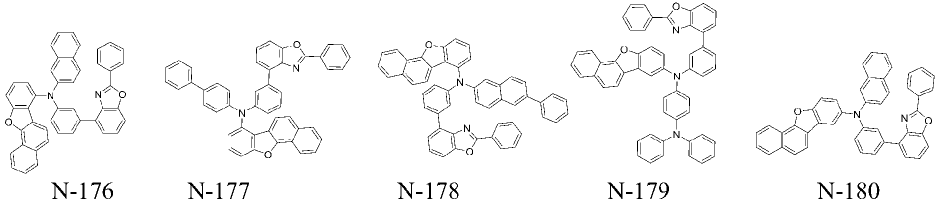

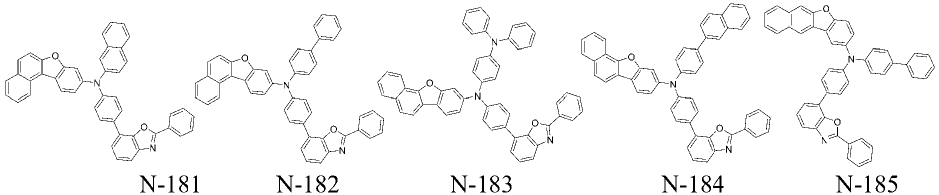

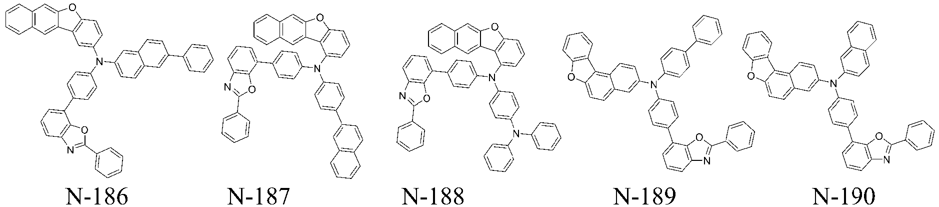

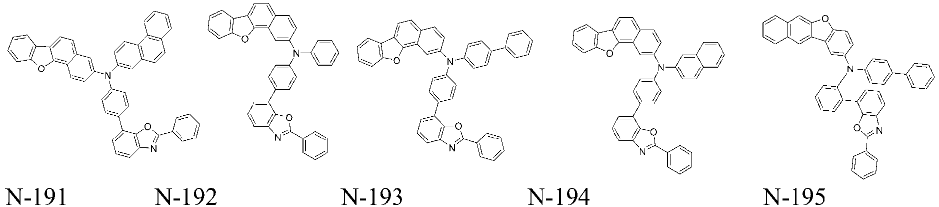

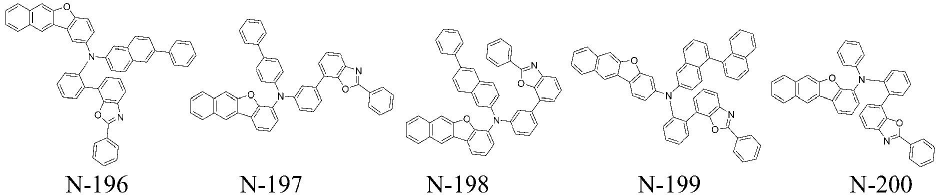

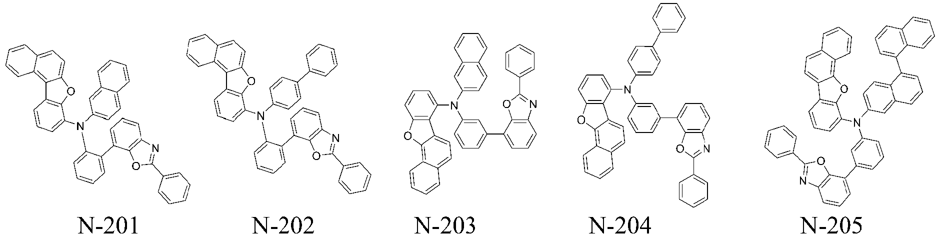

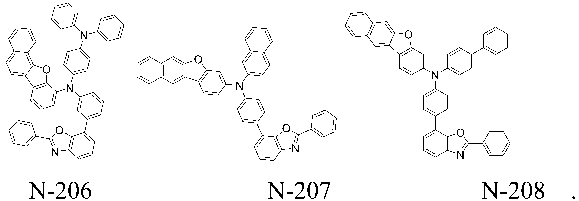

7. A luminescent host material, comprising the benzonaphthofuran-containing oxazole organic compound according to any one of claims 1 to 6.

8. The luminescent host material according to claim 7, wherein the luminescent host material comprises a first host material and a second host material, wherein the first host material is the benzonaphthofuran-containing oxazole organic compound according to any one of claims 1 to 6; the second host material is an organic electroluminescent compound having a structure represented by Formula (2): 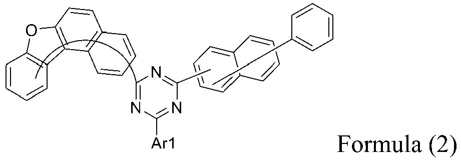
in Formula (2), Ar1 is selected from the group consisting of substituted or unsubstituted C6-C60 aryl, and substituted or unsubstituted C3-C60 heteroaryl;
the substituents in the substituted C6-C60 aryl and the substituted C3-C630 heteroaryl are each independently selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof;
optionally, Ar1 is selected from the group consisting of substituted or unsubstituted C6-C60 non-fused aryl, and substituted or unsubstituted C3-C60 non-fused heteroaryl;
the substituents in the substituted C6-C60 non-fused aryl and the substituted C3-C60 non-fused heteroaryl are each independently selected from the group consisting of deuterium, halogen, cyano, C1-C12 alkyl, C3-C12 cycloalkyl, C6-C30 aryl, C3-30 heteroaryl, C6-C60 arylamine and C3-C60 heteroarylamine, or a combination of at least two thereof;
optionally, Ar1 is selected from the group consisting of substituted or unsubstituted C6-C20 non-fused aryl, and substituted or unsubstituted C3-C20 non-fused heteroaryl;
the substituents in the substituted C6-C20 non-fused aryl and substituted C3-C20 non-fused heteroaryl are each independently selected from the group consisting of deuterium, halogen, cyano, C1-C12 alkyl, C3-C12 cycloalkyl, C6-C30 aryl, C3-C30 heteroaryl, C6-C60 arylamine and C3-C60 heteroarylamine, or a combination of at least two thereof;
optionally, Ar1 is selected from substituted or unsubstituted A group, and the A group is selected from the group consisting of phenyl, biphenyl, and triphenyl;
the substituent in the substituted A is selected from the group consisting of deuterium, phenyl and naphthyl; and
optionally, Arl is selected from the group consisting of phenyl, biphenyl, triphenyl and naphthylphenyl.

9. The luminescent host material according to claim 7 or 8, wherein a mass ratio of the first host material to the second host material is 9:1-1:9;
optionally, a mass ratio of the first host material to the second host material is 2:8-8:2;
more optionally, a mass ratio of the first host material to the second host material is 3:7-7:3;
further optionally, a mass ratio of the first host material to the second host material is 4:6-6:4.

10. An organic electroluminescent material, comprising the benzonaphthofuran-containing oxazole organic compound according to any one of claims 1 to 6, or the luminescent host material according to any one of claims 7 to 9.

11. An organic electroluminescent device, wherein the organic electroluminescent device comprises a cathode, an anode, and an organic layer disposed between the cathode and the anode, wherein the organic layer comprises the benzonaphthofuran-containing oxazole organic compound according to any one of claims 1 to 6, the luminescent host material according to any one of claims 7 to 9, or the organic electroluminescent material according to claim 10;
optionally, the organic layer comprises one or more of a hole injection layer, a hole transport layer, a light-emitting layer, an electron transport layer, and an electron injection layer,
optionally, the hole transport layer comprises the benzonaphthofuran-containing oxazole organic compound according to any one of claims 1 to 6, the luminescent host material according to any one of claims 7 to 9, or the organic electroluminescent material according to claim 10;
optionally, the light-emitting layer comprises the benzonaphthofuran-containing oxazole organic compound according to any one of claims 1 to 6, the luminescent host material according to any one of claims 7 to 9, or the organic electroluminescent material according to claim 10.

12. Use of the organic electroluminescent device according to claim 11 in fiber optic equipment, lighting equipment, electronic photographic photosensitive equipment, photoelectric converter, organic solar cell, switching element equipment, organic light-emitting field-effect transistor, image sensor, or dye laser.
